# EUROPEAN PATENT SPECIFICATION

(11) **EP 1 352 247 B1**
(45) Date of publication and mention of the grant of the patent: **26.10.2005**
(21) Application number: 01959274.0
(22) Date of filing: 26.07.2001
(51) Int. Cl.: G01N 33/68

(54) **ASSAY METHODS FOR IDENTIFYING COMPOUNDS WHICH MAY PROTECT STRATIFIED SQUAMOUS EPITHELIUM AGAINST DAMAGE BY NOXIOUS SUBSTANCES**
VERFAHREN ZUR IDENTIFIZIERUNG VON VERBINDUNGEN DIE DAS MEHRSCHICHTIGE SQUAMOSUS EPITHELIUM GEGEN SCHÄDLICHE SUBSTANZEN SCHÜTZEN
METHODES D'ANALYSE PERMETTANT D'IDENTIFIER DES COMPOSES SUSCEPTIBLES DE PROTEGER L'EPITHELIUM PAVIMENTEUX STRATIFIE CONTRE LES DOMMAGES CAUSES PAR DES SUBSTANCES NOCIVES

(30) Priority: 28.07.2000 US 626196
(43) Date of publication of application: 15.10.2003
(73) Proprietor: The Administrators of the Tulane Educational Fund, New Orleans LA 70112-2699 (US)
(72) Inventor: TOBEY, Nelia, A., River Ridge, LA 70123 (US); ORLANDO, Roy, C., New Orleans, LA 70130 (US)
(74) Representative: Oxley, Rachel Louise
(86) International application number: PCT/US2001/023717
(87) International publication number: WO 2002/010767

(56) References cited:
- US-A- 5 374 537
- NATSUGOE S ET AL: "EXPRESSION OF DESMOGLEIN I IN SQUAMOUS CELL CARCINOMA OF THE ESOPHAGUS" JOURNAL OF SURGICAL ONCOLOGY, NEW YORK, NY, US, vol. 57, no. 2, 1 October 1994 (1994-10-01), pages 105-110, XP000674529
- SCHAEFER S ET AL: "IMMUNOLOGICAL IDENTIFICATION AND CHARACTERIZATION OF THE DESMOSOMAL CADHERIN DSG2 IN COUPLED AND UNCOUPLED EPITHELIAL CELLS AND IN HUMAN TISSUES" DIFFERENTIATION, SPRINGER VERLAG, DE, vol. 60, no. 2, 1 May 1996 (1996-05-01), pages 99-108, XP000674533 ISSN: 0301-4681
- DATABASE SWALL [Online] retrieved from EBI, accession no. CAD1-HUMAN Database accession no. p12830 XP002235753
- DATABASE SWALL [Online] retrieved from EBI, accession no. DSG1-HUMAN Database accession no. Q02413 XP002235754
- PARRISH ALAN R ET AL: "Chemically induced oxidative stress disrupts the E-cadherin/catenin cell adhesion complex." TOXICOLOGICAL SCIENCES, vol. 51, no. 1, 1999, pages 80-86, XP009007580 ISSN: 1096-6080
- PODOLSKY, D. K.: "Mechanisms of regulatory peptide action in the gastrointestinal tract: trefoil peptides." JOURNAL OF GASTROENTEROLOGY, vol. 35, no. suppl. 12, 2000, pages 69-74, XP009007579 Japan

## Description

### TECHNICAL FIELD

The field of this invention relates to assay methods for the identification of compounds which may protect stratified squamous epithelial tissue against injury by noxious substances. More specifically, the field of the invention includes methods for identifying compounds which may be useful in the treatment of conditions associated with gastric reflux, more particularly, diseases such as gastroesophageal reflux disease (GERD).

### BACKGROUND ART

### Gastroesophageal Reflux Disease

Gastroesophageal reflux (GER) is the effortless movement of gastric contents from the stomach to the esophagus. It is a physiologic process, occurring in everyone, many times a day throughout life and without symptoms or signs of tissue injury. However, beginning at about 35-40 years old, GER is increasingly associated with the symptom of heartburn and morphologic injury to the esophagus. Indeed, a Gallup poll reported that up to 44% of adult Americans experience heartburn at least monthly and up to 10% have heartburn daily. The proximate cause for the transition from GER to gastroesophageal reflux disease (GERD) is the ability of noxious materials from the stomach to contact the esophagus with sufficient duration to result in damage to the epithelium. Moreover, it is well established that the major iniurious substance within the refluxate is gastric acid, though the latter may be aided by the presence of pepsin within the refluxate.

The damage resulting from acid exposure can be demonstrated morphologically and/or physiologically by changes in potential difference (PD), electrical resistance (R), or permeability to ions and molecules (Chung, R.S. *et al*. (1975) *Am. J. Physiol*. 229(2):496-500; Dodds, W.J. *et al*. (1970) *Invest Radiol* 5:209-219; Harmon, J.W. *et al*. (1981) *Dig Dis Sci* 26:65-72; Kidder, J.W. *et al*. (1983) *J Lab Clin Med* 102:477-486; Kivilaakso, E. *et al*. (1980) *Surgery* 87:280-285; Salo, J. *et al*. (1982) *Surgery* 92:61-68). In esophageal epithelium the decline in R, in the absence of extensive tissue necrosis, is a direct reflection of increases in paracellular permeability as evidenced by a correlation between the decline in R with an increase in mannitol flux (Orlando, R.C. *et al* (1981) *J Clin Invest* 68:286, Orlando, R.C. *et al* (1984) *Am J Physiol* 246:G718-G724).

The pathophysiology of acid injury has been most thoroughly explored using the rabbit esophageal epithelium as a model for the human esophageal epithelium. Both human and rabbit esophageal epithelia are Na⁺-absorbing 'electrically-tight' stratified squamous epithelia. Each junctional complex consists of tight junctions with a few strands and an intercellular glycoprotein matrix. Moreover, rabbit and human esophageal epithelia both respond to high luminal acidity with a time-dependent biphasic change in transepithelial PD. Further, individual squamous cells utilize a basolateral membrane, acid-extruding, Na⁺/H⁺ exchanger (NHE-1 isotype) as a principal mechanism for regulation of intracellular pH (pHi) (Orlando, R.C. *et al*. In: Castell, D.O. *et al*., eds. The Esophagus (3rd Edition). Philadelphia: Lippincott Williams & Wilkins, 1999,409-419, and Orlando, *R.C., et al*. In: Orlando, *R.C., et al*., eds. Gastroesophageal Reflux Disease. Marcel Decker, *in press*).

Key observations in humans that support a similar pathogenesis for acid reflux-induced injury to human esophageal epithelium as defined in the rabbit model are: 1) luminal perfusion *in vivo* with high concentrations of HCl (Bernstein test) produces a similar biphasic pattern in esophageal PD (Orlando, R.C. *et al*. (2000) *Am. J. Med*. 108 (4A):104S-108S, and Orlando, R.C. *et al*. In: Allen, *A. et al*., eds. Mechanisms of Mucosal Protection in the Upper Gastrointestinal Tract. New York: Raven Press, 1984, 75-79); and, 2) patients with both erosive and nonerosive GERD have the same morphologic hallmark of early acid damage to esophageal epithelium, *i. e.* dilated intercellular spaces. Tobey, N.A. *et al*. (1996) *Gastroenterology* 111:1200-1205.

Previous work has shown that sodium sulfate has a cytoprotective effect in acid exposed rabbit esophagus (Tobey, N.A*. et al*. (1986) *Am J Physiol* 251 (*Gastrointest*. *Liver Physiol*. 14):G866-G869). In U.S. Patents Numbers 5,374,537 and 5,189,056 it was alleged that protection of stratified squamous epithelia against damage from noxious luminal substances, *e*.*g*. HCl, was afforded by chemical compounds having one of the following reactive groups in their molecule: X-SO₃⁻, where X represents oxygen or carbon, and XO₄⁼ or X₂O₇⁼, where X represents an element from Group VIb of the periodic table or sulfur. The compounds which were shown to provide protection against injury to stratified squamous epithelia included 4-acetamido-4'-isothiocyano-2,2'-stilbene disulfonate (SITS), 8-anilino-naphthalene-1-sulfonate (ANS), dinitrodisulfonic acid stilbene (DNDS), sulfonazo III, 4,4'-diisothiocyano-2,2'-stilbene disulfonate (DIDS), bromophenol blue, ethane disulfonate, 1,3-benzene disulfonate, sucrose octasulfate (SOS), dextran sulfate, sodium chromate, sodium dichromate, sodium molybdate, sodium tungstate and sodium sulfate. Labeaga *et al*. disclose dosmalfate as a gastrointestinal and esophageal cytoprotective agent (Labeaga, L. *et al*. (2000) Drugs of Today 2000, Vol. 36, Suppl. A:59-66). Sulfate ester compounds useful for treating and/or preventing gastroesophageal reflux disease (GERD) are disclosed in pending U.S. Provisional Patent Application titled "Methods For Protection Of Stratified Squamous Epithelium Against Injury By Noxious Substances And Novel Agents For Use Therefor" to Hudson *et al*., including disodium 3-(4-isothiocyanatophenyl)-3 phenoxypropane-1,2-disulfate (CDDD-1192) and disodium 3-(4-isothiocyanatophenoxy)-3-(4-isothiocyanatophenyl)propane-1,2-disulfate (CDDD-1193).

*In vitro* methods for identification of potential agents useful for protection of stratified squamous epithelia against damage from noxious substances are disclosed in U.S. Patent Nos. 5,374,537 and 5,189,056, Tobey, N.A. *et al* (1986) *Am J Physiol* 251:G866-G869, and Orlando, R.C. *et al* (1987) *Gastroenterology* 93:352-361.

There is a continued need for agents for treating gastroesophageal reflux disease (GERD) and other conditions associated with the exposure of stratified squamous epithelial tissue to noxious substances. There is a corresponding need for methods for identification of compounds which may protect stratified squamous epithelial tissue from injury resulting from exposure to noxious substances.

### E-cadherin

E-cadherin belongs to the classical cadherin family of proteins, a subfamily of the cadherin superfamily of proteins. Classical cadherins are characterized as Ca²⁺ dependent glycosylated proteins consisting of an N-terminal extracellular domain, a single transmembrane domain and C-terminal cytosolic domain. Plott, R.T. *et al*. (1994) *J Invest Dermatol* 103:168-172. Cadherins are known to mediate cell-cell interactions, such as adhesion. E-cadherin in particular is implicated in the defense of cells against invasive mechanisms, such as in neoplasia, as well as playing an important role in embryonic development and tissue morphogenesis. Bussemakers, M.J. *et al*. (1993) *Molec Biol Reports* 17:123-128; Swami, S. *et al*. (1995) *Am J Gastroenterology* 90:1808-1813; Takeichi, M. (1991) *J Cell Biol* 75:464-474.

The polynucleotide encoding human E-cadherin has been isolated and characterized. Human E-cadherin cDNA has an open reading frame of 882 amino acids **(SEQ ID NO:1)** and a 3' non-coding region of 2035 nucleotides, and has an overall homology with mouse E-cadherin of 78%, and with the chicken E-cadherin related sequence, L-CAM, of 65%. There are few evident evolutionary changes across species, and those that are present are conservative amino acid substitutions. The transmembrane domain of the human and mouse proteins are identical, and the cytoplasmic domain, important for the regulation of the cell-cell binding function of the extracellular domain, is almost identical (with only conservative amino acid substitutions). Bussemakers, M.J. *et al*. (1993) *Molec Biol Reports* 17:123-128.

Processing of the human E-cadherin precursor results in a mature protein with amino acids numbered from 155 to 882. Analysis of human E-cadherin suggests an extracellular domain of approximately 553 amino acids (amino acid 155 to approximately amino acid 707) with five internally repeated domains of approximately 110 amino acids which are involved in Ca²⁺ binding. The corresponding nucleotide bases for the extracellular domain are from approximately nucleotide 557 to approximately nucleotide 2215 (nucleotide numbering according to Bussemakers, M.J. *et al*. (1993) *Molec Biol Reports* 17:123-128). The five extracellular repeat domains (EC1-EC5) have been reported to correspond to amino acid residues approximately: EC1 - 155-262; EC2 - 263-375; EC3 - 376-486; EC4 - 487-593; EC5 - 594-697, according to the sequence reported in GenBank accession number P12830 (http://www.ncbi.nlm.nih.gov/PubMed/). EC1-EC4 have from 19-29% internal sequence identity, while EC5 is less related to the other four subdomains with only 10-16% sequence identity. Alattia, J.R. *et al*. (1999) *Cell Mol Life Sci* 55:359-367.

Several structural studies of isolated portions of the extracellular domain of E-cadherin have also been carried out, including X-ray crystallography and NMR studies. Alattia, J.R. *et al*. (1999) *Cell Mol Life Sci* 55:359-367 and references cited therein; Overduin, M. (1996) *J Biol NMR* 7:173-189; Gumbiner, B.M. *et al*. (1996) *Cell* 84:345-357. The basic structure of the intra-domain repeats appear to be a fold consisting of a seven stranded β-sheet with the N and C termini of the fragment at opposite ends of the fold. The fold resembles a barrel and contains two small helices. It is thought that the N-terminal tip of the molecule is involved in cell adhesion, with the adhesion specificity residing in the HAV region in domain 1 of E-cadherin. Overduin, M. *et al*. (1995) *Science* 267:386-389; Takeichi, M. (March 22, 1991) *Science* 251:1451-1455; Pertz, O. *et al*. (1999) *EMBOJ* 18:1738-1747; Geiger *et al*. (1992) *Ann Rev Cell Biol* 8:307-332.

E-cadherin can also be characterized by its Ca²⁺ dependency. In the presence of Ca²⁺, E-cadherin is resistant to cleavage by trypsin, while in the absence of Ca²⁺, the protein can be cleaved by trypsin, resulting in an approximately 80kD fragment, which corresponds to the extracellular domain. These findings are consistent with the presence of Ca²⁺ binding regions in the extracellular domain. Plott, *R.T. et al*. (1994) *J Invest Dermatol* 103:168-172; Gumbiner, B.M. *et al*. (1986) *J Cell Biol* 102:457-468.

Cadherins have been identified as the major adhesion receptors of the zonula adherens junctions of epithelia. In addition, the expression and activity of E-cadherin is vital for the tight association of cells and the maintenance of the macromolecular structure of epithelial tissue. In the absence of functional E-cadherin, intercellular adhesion is not maintained, despite the presence of other adhesion and junctional proteins. Gumbiner, B.M. (1996) *Cell* 84:345-357. Calcium also appears necessary for cell-cell adhesion. Alattia, J.R. *et al* (1999) *Cell Mol Life Sci* 55:359-367.

Studies of kidney epithelial tissue reveal that antibodies which can bind E-cadherin also inhibit the recovery of R injunctions made permeable by the removal of Ca²⁺ from the surrounding solution. The antibody recognizes a 118 kD polypeptide from kidney epithelium that is the same as or related to the cell adhesion molecule Uvomorulin/E-cadherin. Additionally, after the junction has been made permeable in the absence of Ca²⁺, incubation with trypsin leads to poor recovery of R upon the restoration of Ca²⁺. The lack of recovery in these cells compared to cells not treated with trypsin or cells treated with trypsin in the presence of Ca²⁺, without concomitant loss of cells, suggests that the lack of recovery is due to the degradation by trypsin of a cell-surface protein. Gumbiner, B.M. *et al*. (1986) *J Cell Biol* 102:457-468. The antibody isolated in the experiments described above also recognized the approximately 80kD fragment of E-cadherin resulting from E-cadherin cleaved by trypsin in the presence of Ca²⁺.

### Desmosomal Subfamily

Desmocollin 3, which belongs to the desmosomal subfamily of the cadherin superfamily, is a 896 residue protein, with the extracellular domain having 555 amino acids numbered 136-690, as described in GenBank accession number Q14574 **(SEQ ID NO:5).**

The desmogleins, a subfamily of the desmosomal cadherins, share amino acid homologies in both the cytoplasmic and extracellular domains with the classical cadherins. Desmoglein 1 is a 1049 residue protein, with the extracellular domain having 499 amino acids numbered 50-548, as described in GenBank accession number IJHUG1 **(SEQ ID NO:2)** (http://www.ncbi.nlm.nih.gov/PubMed/). Desmoglein 2 is a 1117 residue protein, with the extracellular domain having 560 amino acids numbered 49-608, as described in GenBank accession number Q14126 **(SEQ ID NO:3).** Desmoglein 3 (also known as pemphigus vulgaris antigen) is a 999 residue protein, with the extracellular domain having 566 amino acids numbered 50-615, as described in GenBank accession number IJHLTG3 **(SEQ ID NO:4).** Desmoglein 3 has been shown to have an overall sequence identity of 33% to human E-cadherin. Plott, R.T. *et al*. (1994) *J Invest Dermatol* 103:168-172. Additionally, alignment of the human E-cadherin sequence with extracellular subdomains 1-5 of desmoglein 3 result in sequence identities between the two proteins of EC1, 31%, EC2, 45%, EC3, 33%, EC4, 30% and EC5, 28%. Plott, R.T. *et al*. (1994) *J Invest Dermatol* 103:168-172.

### Other Polypeptides With Substantial Homology to E-cadherin

The HA(V/N) region of HA1 chain influenza strain A hemagglutinins show substantial sequence homology with cadherins. Blaschuk *et al*. (1990) *J Mol Biol* 211:679-682. The hemagglutinins examined were split into two groups, with the overall HA(V/N) domain of group 1 hemagglutinins **(SEQ ID NOS:6-13)** having 25% of residues that are identical to the consensus sequence for the cadherin family, and an additional 25% of residues being either partially conserved or homologous according to the Dayhoff matrix. For group 2 hemagglutinins **(SEQ ID NOS:14-21)** the percent identity and homology are 13% and 30%, respectively.

HAV domains of fibroblast growth factor receptors (FGF-R) **(SEQ ID NOS:22-25)** showed sequence identity of 25% with cadherins with an additional 33% being partially conserved or homologous. Byers, S. *et al*. (1992) *Dev Biol* 152:411-414.

### DISCLOSURE OF THE INVENTION

One method of the present invention comprises assaying for a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted within the extracellular domain of **SEQ ID NO:1;** and b) determining whether the compound binds to the polypeptide; wherein binding of the compound to the polypeptide indicates that the compound may protect stratified squamous epithelia from damage by a noxious substance.

Another method of the present invention comprises assaying for a compound which may protect stratified squamous epithelia from damage by a noxious substance, comprising, in order: a) contacting a polypeptide with a labeled cytoprotective agent; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a compound; and c) comparing binding of the agent to the polypeptide contacted with the compound to a control conducted where the polypeptide is contacted with the agent and is not contacted with the compound; wherein a decrease in binding of the agent to the polypeptide contacted with the compound compared to the control indicates that the compound may protect stratified squamous epithelia against damage by a noxious substance.

Another method of the present invention comprises identifying a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a monoclonal antibody to the polypeptide; and c) comparing the amount of antibody binding to the polypeptide contacted with the compound to the amount of antibody binding to a control; wherein a decrease in the amount of antibody binding to the polypeptide which has been contacted with the compound compared to the control indicates that the compound may protect stratified squamous epithelia from damage by a noxious substance.

Another method for use in the present invention comprises assaying for a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted with in the extracellular domain of **SEQ ID NO:1;** b) determining the stability of the polypeptide; and c) comparing the stability of the polypeptide contacted with the compound to the stability of a control; wherein a greater stability for the polypeptide contacted with the compound compared to the control indicates that the compound may protect stratified squamous epithelia from damage by a noxious substance.

Another method for use in the present invention comprises assaying for a compound which may protect stratified squamous epithelia from damage by a noxious substance, comprising, in order: a) contacting a polypeptide with the compound; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a noxious substance; and c) determining the stability of the polypeptide; and d) comparing the stability of the polypeptide contacted with the compound and the noxious substance to the stability of a control; wherein if the stability of the polypeptide contacted with the compound and noxious substance is greater than that of the control, the compound may protect stratified squamous epithelium against damage by the noxious substance.

An additional method of the present invention comprises assaying for a compound which may protect stratified squamous epithelia from damage by a noxious substance, comprising: a) contacting a polypeptide with the compound; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a noxious substance; c) determining the amount of fragmentation of the polypeptide; and d) comparing the amount of fragmentation of the polypeptide to a control; wherein a decrease in the amount of fragmentation of the polypeptide contacted with the compound compared to the control indicates that the compound may protect stratified squamous epithelia from damage by a noxious substance.

### MODES FOR CARRYING OUT THE INVENTION

### Definitions

As used herein, the term "compound" means a biological or chemical compound such as a simple or complex organic or inorganic molecule, a peptide, a protein or an oligonucleotide. A vast array of compounds can be synthesized, for example oligomers, such as oligopeptides and oligonucleotides, and synthetic organic compounds based on various core structures, and these are also included in the term "compound". In addition, various natural sources can provide compounds for screening, such as plant or animal extracts, and the like. Compounds can be tested singly or in combination with one another. "Compounds" may also be referred to as "drugs" or "pharmaceutical compounds" or "pharmaceutical candidates".

A "noxious substance", as used herein, refers to a substance which causes injury to stratified squamous epithelium *in vivo* or *in vitro.* Examples of such "noxious substances" include acids or other substances, including, but not limited to, gastric acid, HCl, N-acetylcysteine, pepsin, acid-pepsin, gastrointestinal fluid, or other irritant which contacts epithelial tissue. The term "noxious substance" may also be used to refer to a combination of noxious substances, such as two or more noxious substances.

An "individual" is a vertebrate, preferably a mammal, more preferably a human. Mammals include, but are not limited to, farm animals, sport animals, primates, rodents and pets, including pigs, sheep, horses, cows, rabbits, mice and rats.

A "susceptible individual" is an individual who suffers from or is likely to or predisposed to suffer from a condition(s) which causes contact or exposure between their epithelial tissues and noxious substances. In humans these conditions may include, for example, gastroesophageal reflux disease (GERD), heartburn, indigestion, laryngitis, Barrett's Esophagus, or pharyngitis. In animals these conditions may include, for example, peptic ulcers, gastritis, erosions, perforation and hemorrhage.

The terms "injury" or "damage" as used herein are interchangeable and refer to the cellular and morphological manifestations and symptoms resulting from exposure to or contact with a noxious substance. Indications of injury or damage include, but are not limited to, acid-induced decline in PD, acidification of the intracellular or intercellular space, prominently dilated intercellular spaces, increased permeability of intercellular junctions to noxious substances; macroscopic erosions and ulceration of epithelial tissue, cell edema, inflammation and necrosis, peptic stricture, Barrett's Esophagus, heartburn, laryngitis, pharyngitis, hoarseness and sore throat.

The terms "stratified squamous epithelium", "stratified epithelium", "epithelia" and "epithelium" are interchangeable as used herein. Sources of stratified squamous epithelium include, for example, buccal, oropharyngeal, esophageal and laryngeal epithelium, rumen and forestomach.

The polypeptide known as "E-cadherin" is also known as "CAM 120/80", "L-CAM", "Arc-1" and "Uvomorulin". Unless otherwise noted, these terms may be used interchangeably herein. A preferred form of E-cadherin is the human sequence.

The term "cadherin" refers to a protein which is a member of the cadherin superfamily of proteins. The cadherin superfamily includes, for example, the subfamily of classical cadherins, specific examples of which include E-cadherin, N-cadherin, and P-cadherin, and the subfamily of desmosomal proteins, specific examples of which include desmogleins 1, 2 and 3, and desmocollin 3. Natural sources of cadherins may be found in vertebrates, including humans, farm animals, sport animals, primates, rodents and pets, for example, chickens, pigs, sheep, horses, cows, rabbits, mice and rats.

The term "extracellular domain" refers to the region of a protein that is not embedded in the membrane and exists outside the cellular membrane.

The term "extracellular portion" refers to an isolated polypeptide comprising at least 5 amino acid residues of an extracellular domain of a protein. For example, the term "extracellular portion" may encompass smaller domains or subdomains, such as the 5 repeat regions of approximately 110 residues within the extracellular domain of E-cadherin. The isolated polypeptide may be purified from a natural source or maybe obtained by synthetic methods known in the art.

A "cytoprotective agent" is an agent which protects stratified squamous epithelia from damage by a noxious substance. Classes of compounds which include examples of such agents include sulfonates, sulfate esters and inorganic tetrahedral-shaped divalent oxyanions. Specific examples include SITS, SOS, CDDD-1192 and CDDD-1193.

The term "protection" as used herein refers to the reduction of damage by a noxious substance to epithelium which has been contacted with an agent *in vivo* or *in vitro*. The protection provided by the agent may last for at least 10 minutes, at least 20 minutes, at least 30 minutes; at least 1 hour, at least 2 hours, at least 4 hours, at least 8 hours, at least 12 hours, at least 24 hours after contact with the compound.

The term "irreversible protection" refers to protection afforded by compounds, which, when contacted with the epithelium, are resistant to being washed off the epithelium. Irreversible protection may be indicated by compounds, which for example, when contacted with the epithelium *in vitro*, continue to prevent or reduce acid-induced decline in R after at least one washing with a buffer such as normal Ringer solution.

The term "control" refers to an experiment conducted under the same conditions as the experiment to which it is being compared and is designed to measure the same experimental parameter. "Controls" may be used to establish typical values for the experimental parameter being measured under particular conditions. A "control" is not required to be run in parallel with the experiment in question and may be run within hours, days, weeks or months of the experiment in question.

An "antibody" (interchangeably used in plural form) is an immunoglobulin molecule capable of specific binding to a target, such as a polypeptide, through at least one antigen recognition site which is located in the variable region of the immunoglobulin molecule. An antibody can be from any source of animal capable of producing them, for example, mouse, rat, rabbit, primate, or human antibodies. As used herein, the term encompasses not only intact antibodies, but also fragments thereof (such as Fab, Fab', F(ab')₂, Fv, single chain (ScFv)), mutants thereof, fusion proteins, chimeric antibodies, humanized antibodies, and any other modified configuration of the immunoglobulin molecule that comprises an antigen recognition site of the required specificity. The term "antibody" includes polyclonal antibodies and monoclonal antibodies.

The terms "polypeptide", "oligopeptide", "peptide" and "protein" are used interchangeably herein to refer to polymers of amino acids of any length. The polymer may be linear or branched, it may comprise modified amino acids, and it may be assembled into a complex of more than one polypeptide chain. The terms also encompass an amino acid polymer that has been modified naturally or by intervention; for example, by disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

In the context of polypeptides, a "linear sequence" or a "sequence" is an order of amino acids in a polypeptide in an N-terminal to C-terminal direction in which residues that neighbor each other in the sequence are contiguous in the primary structure of the polypeptide. A "partial sequence" is a linear sequence of part of a polypeptide which is known to comprise additional residues in one or both directions.

An "isolated" or "purified" polynucleotide, polypeptide, antibody or cell is one that is substantially free of the materials with which it is associated in nature. By substantially free is meant at least 50%, preferably at least 70%, more preferably at least 80%, and even more preferably at least 90%, at least 95%, at least 99% free of the materials with which it is associated in nature. As used herein, an "isolated" polynucleotide or polypeptide also refers to recombinant polynucleotides or polypeptides, which, by virtue of origin or manipulation: (1) are not associated with all or a portion of a polynucleotide or polypeptide with which it is associated in nature, (2) are linked to a polynucleotide or polypeptide other than that to which it is linked in nature, or (3) does not occur in nature, or (4) in the case of polypeptides arising from expression of recombinant polynucleotides. Thus, for example, an isolated substance may be prepared by using a purification technique to enrich it from a source mixture. Enrichment can be measured on an absolute basis, such as weight per volume of solution, or it can be measured in relation to a second, potentially interfering substance present in the source mixture. Increasing enrichments of the embodiments of the compositions used in the methods of this invention are increasingly more preferred. Thus, for example, a 2-fold enrichment is preferred, 10-fold enrichment is more preferred, 100-fold enrichment is more preferred, 1000-fold enrichment is even more preferred. A substance can also be provided in an isolated state by a process of artificial assembly, such as by chemical synthesis or recombinant expression.

A substance is said to be "selective" or "specific" if it reacts or associates more frequently, more rapidly, with greater duration and/or with greater affinity with a particular cell or substance than it does with alternative cells or substances. An antibody "specifically binds" to a target if it binds with greater affinity, avidity, more readily, and/or with greater duration than it binds to other substances.

As used herein, "naturally occurring," "native," or "wild type" refers to endogenous E-cadherin polynucleotides and the E-cadherin protein(s) expressed thereby, including alleles and allelic forms of the protein(s). These terms include full-length and processed polynucleotides and polypeptides. Processing can occur in one or more steps, and these terms encompass all stages of processing. For instance, polypeptides having or lacking a signal sequence may be used. "Non-naturally occurring", "non-native", or "non-wild type" refer to all other E-cadherin polynucleotides and polypeptides.

The term "stability" refers to the measure of the ability of a protein or fragment thereof to maintain its native conformation and/or activity. Stability may be measured by techniques well known in the art.

A polypeptide or polypeptide region having a certain percentage (for example, 10%, 13%, 20%, 50%, 70%, 75%, 80%, 85%, 90%, or 95%) of "sequence identity" to another sequence means that, when aligned, that percentage of amino acids are the same in comparing the two sequences. This alignment and percent identity may be determined using software programs known in the art, for examples those described in *Short Protocols in Molecular Biology* (John Wiley and Sons., eds., 1999).

Sequence identity is generally determined after sequences have been aligned to maximize the number of matches between the sequences. A variety of alignment programs and algorithms are known in the art. One suitable program for polypeptide sequences is blastn which uses a heuristic search algorithm (BLAST) that is also useful for sequence alignments. See, for example, Altschul, S.F. *et al*. (1990) *J Mol Biol* 215:403-410. BLAST programs may be accessed through the National Center for Biotechnology Information (NCBI) intemet site (www.ncbi.nlm.nih.gov/BLAST/). The default parameters of the NCBI blastn programs are suitable for generating sequence alignments for the sequences described herein. Other suitable alignment programs are commercially available. The GAP program from Genetics Computer Group, Inc. (GCG; Madison, WI), for example, uses the algorithm of Needleman and Wunsch (1970, *J Mol Biol* 48:443-453) to generate the alignment of two complete sequences that maximizes the number of matches and minimizes the number of gaps. For another example, the BESTFIT program from GCG uses the local homology algorithm of Smith and Waterman (1981, *Advances in Applied Mathematics* 2:482-489) to generate the optimal alignment of the best segment similarity between two sequences through the insertion of gaps to maximize the number of matches. When identifying the best alignment, the default gap penalty is 50 and the default gap extension penalty is 3 for the GAP and BESTFIT programs.

Chemical terms, unless otherwise defined, are used as known in the art.

"A", "an" and "the" include plural references unless the context clearly dictates otherwise.

### General Techniques

Techniques for isolation and manipulation of cadherin proteins may be found in, for example, Gumbiner, B.M. (1996) *J Cell Biol*:102:457-468, Gumbiner, B.M. (1996) *Cell* 84:345-357, Takeichi, M. (1977) *J Cell Biol* 75:464-474, and Takeichi, M. (1988) *Development* 102:639-655. Techniques and protocols for manipulating epithelial tissue and using Ussing chambers may be found in the art and in particular in: U.S. Patent No. 5,374,537; U.S. Patent No. 5,189,056; Abdulnour-Nakhoul, S. *et al* (2000) *Am J Physiol* 278:G113-G120, Carney,C.N. *et al* (1981) *Lab Invest* 45:198-208, Orlando, R.C. *et al* (1981) *J Clin Invest* 68:286, Orlando, R.C. *et al* (1984) *Am J Physiol* 246:G718-G724, Khalbuss, W.E. *et al* (1995) 108:662-672, Tobey, *N.A. et al* (2000) *J Invest Med* 108(4A):144A, Tobey, N.A. *et al* (1999) *Gastroenterology* 116:A334, Orlando, R.C. *et al*. (2000) *Am J Med* 108(4A):104S-108S, Tobey, *N.A. et al*. (1989) *Gastroenterology* 96:1466-1477, Orlando, R.C. *et al* (1984) *New York: Raven Press:75-79,* Orlando, R.C. *et al* (1982) *Gastroenterology* 83:1026-1032, and Tobey, N.A. *et al* (1996) *Gastroenterology* 83:1026-1032.

### Methods of Assaying for Compounds Which May Protect Stratified Squamous Epithelia

As described in the following examples, we have identified the mechanism by which cytoprotective agents, such as SITS or SOS, protect epithelial tissue from exposure to noxious substances. Without being bound by any particular theory, it appears that this mechanism comprises the binding of the agent to the extracellular domain, portion, or a region or fragment thereof, of E-cadherin, a junctional protein found in the zonula adherens of epithelial tissue. Based upon this discovery, novel assay techniques have been designed to screen for compounds which will bind to a polypeptide selected from the group consisting of an extracellular portion of a cadherin or a peptide sequence having at least 13% sequence identity to a sequence depicted within the extracellular domain of E-cadherin **(SEQ ID NO:1),** where binding is indicative of a compound which may protect stratified squamous epithelial from damage or injury by noxious substances, such as damage stemming from acid reflux.

Accordingly, one method of the present invention comprises assaying for a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted within the extracellular domain of **SEQ ID NO:1;** and b) determining whether the compound binds to the polypeptide; wherein binding of the compound to the polypeptide indicates that the compound may protect stratified squamous epithelia from damage by a noxious substance.

An additional embodiment further comprises the step of: c) comparing the binding of the compound to the polypeptide with the binding of a cytoprotective agent to the polypeptide; wherein a greater than or equal level of binding by the compound indicates that the compound may protect stratified squamous epithelia from damage by the noxious substance.

Another method of the present invention comprises assaying for a compound which may protect stratified squamous epithelia from damage by a noxious substance, comprising, in order: a) contacting a polypeptide with a labeled cytoprotective agent; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a compound; and c) comparing binding of the agent to the polypeptide contacted with the compound to a control conducted where the polypeptide is contacted with the agent and is not contacted with the compound; wherein a decrease in binding of the agent to the polypeptide contacted with the compound compared to the control indicates that the compound may protect stratified squamous epithelia against damage by a noxious substance.

The determination of protein to substrate binding, in this context, binding to either compound or cytoprotective agent, is well known in the art, and need not be discussed in detail herein. Exemplary techniques for determining binding include, for example, labeling the substrate (*i*.*e*. the compound or agent binding to the protein) with, for example, a radioactive isotope or fluorescent label. Examples of fluorescent labels include, but are not limited to, biotin, fluoroscein, Texas red, Lucifer yellow, and rhodamine. Other labeling methods include electron dense tracers, such as, alkaline phosphatase, horseradish peroxidase and glucose oxidase.

Another method of the present invention comprises identifying a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a monoclonal antibody to the polypeptide; and c) comparing the amount of antibody binding to the polypeptide contacted with the compound to the amount of antibody binding to a control; wherein a decrease in the amount of antibody binding to the polypeptide which has been contacted with the compound compared to the control indicates that the compound may protect stratified squamous epithelia from damage by a noxious substance.

In one embodiment of the above method, the control may comprise contacting the polypeptide with the monoclonal antibody or may comprise contacting the polypeptide with a cytoprotective agent and then contacting the polypeptide with the monoclonal antibody.

Another method for use in the present invention comprises assaying for a compound which may protect stratified squamous epithelia from damage by a noxious substance, comprising, in order: a) contacting a polypeptide with a compound; wherein the polypeptide is selected from the group consisting of an extracellular portion of a cadherin or a peptide sequence having at least 13% sequence identity to a sequence depicted with in the extracellular domain of **SEQ ID NO:1;** b) determining the stability of the polypeptide; and c) comparing the stability of the polypeptide contacted with the compound to the stability of a control; wherein a greater stability for the polypeptide contacted with the compound compared to the control indicates that the compound may protect stratified squamous epithelia from damage by a noxious substance.

In one embodiment, controls for the method described above includes where the control comprising the polypeptide is not contacted with the compound or where the polypeptide is contacted with a cytoprotective agent and is not contacted with the compound.

Another method for use in the present invention comprises assaying for a compound which may protect stratified squamous epithelia from damage by a noxious substance, comprising, in order: a) contacting a polypeptide with the compound; wherein the polypeptide is selected from the group consisting of an extracellular portion of a cadherin or a peptide sequence having at least 13% sequence identity to a sequence depicted within the extracellular domain of **SEQ ID NO:1**; b) contacting the polypeptide with a noxious substance; and c) determining the stability of the polypeptide; and d) comparing the stability of the polypeptide contacted with the compound and the noxious substance to the stability of a control; wherein if the stability of the polypeptide contacted with the compound and noxious substance is greater than that of the control, the compound may protect stratified squamous epithelium against damage by the noxious substance.

Controls for the above method may include, in certain embodiments, where a control comprising the polypeptide is not contacted with the compound but is contacted with the noxious substance or where the polypeptide is contacted with a cytoprotective agent and then contacted with the noxious substance.

While not wishing to be bound by any particular theory, it appears, from the data presented in the Examples, that the mechanism by which cytoprotective agents function is to maintain the stability of the junctional protein E-cadherin in the presence of noxious substances. While the overall stability of the protein is not enhanced by the binding of cytoprotective agents over that of the native protein, under, for example, denaturing conditions or in the presence of a noxious substance, native-like characteristics should be maintained, such as exemplified by conformation or biological activity (e.g. the maintenance of junctional impermeability).

The stability of the extracellular portion may be measured by techniques well known in the art, and need not be discussed herein. Examples of such techniques include, but are not limited to, calorimetric measurement, such as the determination of the temperature of denaturation, for example, digital scanning calorimetry, resistance to denaturation, as measured by, for example, titration against temperature, pH or chemical denaturants such as guanidine hydrochloride or urea. Stability may be measured under varying conditions of hypertonicity, hypotonicity, and concentrations of N-acetylcysteine, EMPIGEN® detergent (Calbiochem, San Diego, CA),bile acids and bile salts. Titrations may be monitored by a number of means, including HPLC (high performance liquid chromatography), FPLC (fast performance liquid chromatography), and spectroscopic or optical means, such as fluorescence. Additional examples of measures of stability include resistance to fragmentation by, for example, acid cleavage or enzymatic digestion, including but not limited to enzymes such as pepsin, trypsin, chymotrypsin and concentrated acids such as hydrochloric acid, sulfuric acid, phosphoric acid, gastric acid and others. Fragmentation upon exposure to noxious substances occurring within the digestive system or testing analogues thereof, is also contemplated. Examples of such substances include acid, acid-pepsin and N-acetylcysteine. Examples of determining the degree of fragmentation are discussed below. Persons of skill in the art will recognize that numerous biophysical techniques are available to measure the stability of proteins, or portions thereof, with or without substrate (*i*.*e*. compound or agent) bound to the protein or protein fragment.

An additional method of the present invention comprises assaying for a compound which may protect stratified squamous epithelia from damage by a noxious substance, comprising: a) contacting a polypeptide with the compound; wherein the polypeptide is selected from the group consisting of an extracellular portion of a cadherin or a peptide sequence having at least 13% sequence identity to a sequence depicted within the extracellular domain of **SEQ ID NO:1;** b) contacting the polypeptide with a noxious substance; c) determining the amount of fragmentation of the polypeptide; and d) comparing the amount of fragmentation of the polypeptide to a control; wherein a decrease in the amount of fragmentation of the polypeptide contacted with the compound compared to the control indicates that the compound may protect stratified squamous epithelia from damage by a noxious substance.

In certain embodiments, controls for the above-described method may include where the polypeptide is not contacted with the compound but is contacted with the noxious substance or where the polypeptide is contacted with a cytoprotective agent and then contacted with the noxious substance.

In the present embodiment, fragmentation is the result of exposure to a noxious substance, such as acid, N-acetylcysteine or acid-pepsin. The degree of fragmentation is indicative of the amount of protection afforded by the compound being assayed, where a decrease in fragmentation compared to a control indicates protection. Determination of the degree or amount of fragmentation may be accomplished using techniques known in the art, exemplary techniques including electrophoresis, including SDS gel electrophoresis and capillary electrophoresis, isoelectric focusing, HPLC, FPLC, mass spectrometry, 2D-gels, amino acid analysis, and amino acid sequencing.

In general, for any of the methods of the present invention, controls are run such that they comprise the same polypeptide under the same experimental conditions, except that controls are not contacted with the compound which is being tested or are instead contacted with a cytoprotective agent, as specified. Controls need not be run in parallel to the experiment in question, and may consist of prior experimentation done days, weeks or months prior to the experiment in question, that establishes typical values for a set of experimental conditions. Examples of particular cytoprotective agents for use in control experiments may be found, for example in U.S. Patent Nos. 5,374,537 and 5,189,056, and in pending U.S. Provisional Patent Application titled "Methods For Protection Of Stratified Squamous Epithelium Against Injury By Noxious Substances And Novel Agents For Use Therefor" to *Hudson et al*. Preferred classes of compounds for use as cytoprotective agents include sulfate esters and sulfonates. Examples of preferred cytoprotective agents includes SITS, SOS and CDDD-1193 and CDDD-1192.

Experimental conditions may include, for example, pH, temperature, duration of contact with a noxious substance, concentration of noxious substance, concentration of polypeptide, number and duration of washes, composition of wash solutions and the like. Preferred experimental conditions include, for example, pH from about 1-14, temperature from about 0-100°C, duration of contact with noxious substances from about 1 minute to 24 hours, duration of washes from about 1 minute to 24 hours; number of washes from about 1 to 20, concentration of noxious substances from about 1 µmolar to 1 molar, concentration of polypeptide from about 1 picomolar to 10 millimolar. More preferred experimental conditions may include, for example, pH from about 1-4, pH from about 1-2.5, pH about 1.6, temperature from about 25-37°C, temperature of about 37°C, duration of contact with noxious substances from about 1 minute to 1 hour, duration of contact with noxious substances from about 1 to 30 minutes, duration of washes from about 1 to 5 minutes, number of washes from about 1 to 5, number of washes from about 3 to 4, concentration of HCl from about 10 millimolar to 100 millimolar, concentration of pepsin from about 0.1 to 10 mg/ml, concentration of bile salts from about 500 µM to 1 mM, concentration of polypeptide from about 1 nanomolar to 10 micromolar, and concentration of polypeptide from about 1 micromolar to 10 millimolar. *In vitro* experiments may be run in physiologic buffers or serum. Wash solutions may be any aqueous vehicle. Preferred examples include water, saline, Ringer, HEPES buffer, Tris-buffer, saliva, or salivary ion composition solutions. The compound or agent may be contacted with the polypeptide, for example, for at least 30 seconds, at least 1 minute, at least 5 minutes, at least 10 minutes, at least 20 minutes, at least 30 minutes, at least 60 minutes, or at least 90 minutes.

Preferred compounds to be assayed include, but are not limited to, compounds selected from the group including sulfonates and sulfate esters.

As discussed in detail in the Examples, we have identified the extracellular domain of the zonula adherens junctional protein E-cadherin as the region where cytoprotective agents bind. In addition, monoclonal antibodies to desmoglein 1 and 2, and desmocollin 3, members of the cadherin superfamily of proteins, appear to bind to the extracellular domain of E-cadherin. While not wishing to be bound by a particular theory, this monoclonal antibody cross-reactivity is likely a result of the degree of homology or identity of the extracellular domains of these cadherin proteins, and suggests that compounds which bind the extracellular domain of E-cadherin will also bind the extracellular domains of similar proteins.

Members of the cadherin superfamily of proteins, and extracellular portions thereof, may be used as polypeptides in the novel assays techniques described herein. Preferred sequences include extracellular portions of proteins of the classical cadherin and desmosomal subfamilies. More preferred are extracellular portions from the proteins E-cadherin **(SEQ ID NO:1),** desmoglein 1 **(SEQ ID NO:2),** 2 **(SEQ ID NO:3),** or 3 **(SEQ ID NO:4),** and desmocollin 3 **(SEQ ID NO:5).** Even more preferred are extracellular portions from E-cadherin. Particularly preferred are extracellular portions from E-cadherin residues 155-262, desmoglein 1 residues 52-157, desmoglein 2 residues 49-159, desmoglein 3 residues 52-157, and desmocollin 3 residues 136-243.

Additional peptides with sequence similarity to extracellular portions of E-cadherin may also be used as polypeptides for testing possible drug candidates or compounds and may be used in any of the methods of the invention described herein. Examples of such peptides include the HA(V/N) region of the HA1 chain of influenza A hemagluttinin, examples of which are shown in **SEQ ID NOS:6-21** and HAV region sequences from fibroblast growth factor receptor, examples of which are shown in **SEQ ID NOS:22-25** (Table 1).

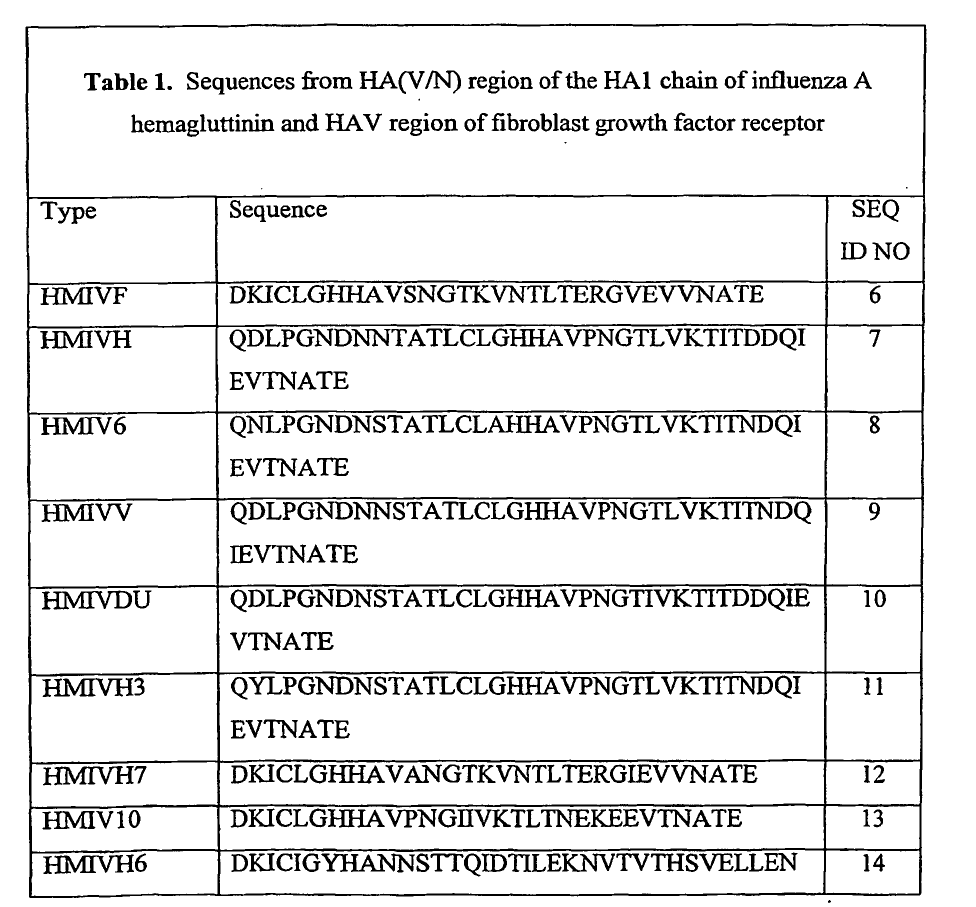

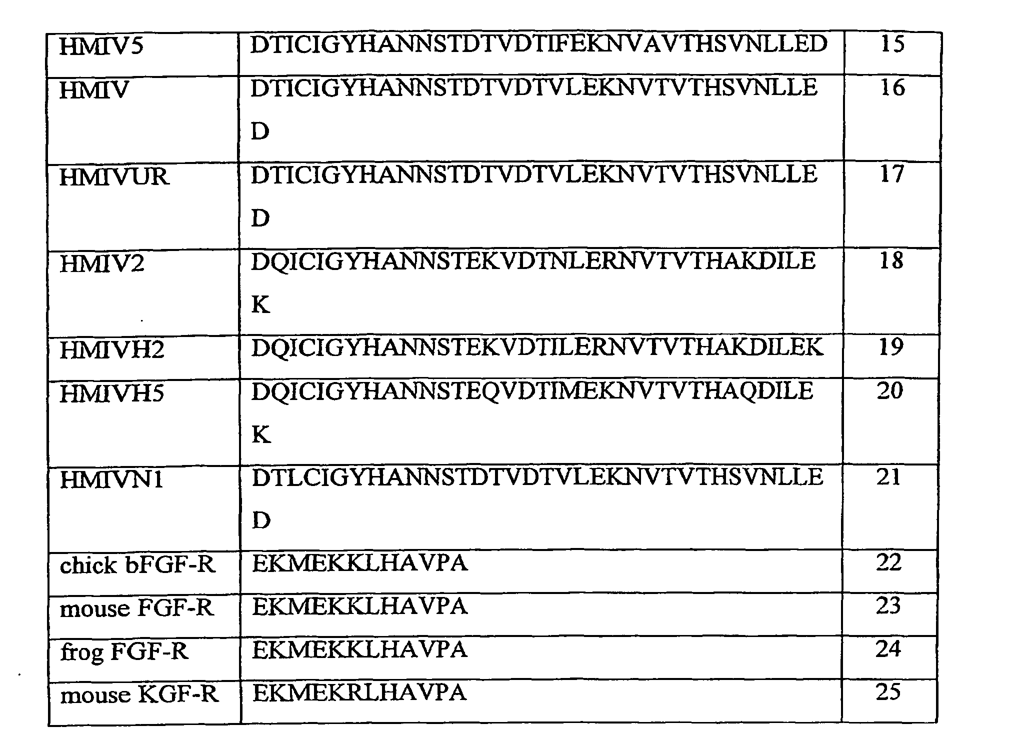

Preferably, the peptide for use in the assay methods described herein will have at least 13%, at least 19%, at least 20%, at least 30%, at least 40%, at least 50%, at least 60%, at least 70% sequence identity with an extracellular portion of E-cadherin as depicted in **SEQ ID NO:1.**

In certain embodiments, the polypeptide may comprise the sequence, or fragment thereof, of the sequences described above, wherein the fragment of the sequence is at least 5, at least 6, at least 10, at least 25, at least 50, at least 100, at least 110, at least 120; at least 150, at least 200, at least 220, or at least 300 amino acids in length.

In a preferred embodiment, the human sequence of the protein will be used. In another embodiment, other variants may be used in the methods of the invention described herein, including those from farm animals, pet, rodents, primates and sport animals, such as chicken, rabbit, rat, mouse, horse, cow, sheep, and pig sequences.

Embodiments of any of the assay methods described above may further comprise the following steps, in order: contacting a sample comprising stratified squamous epithelial tissue with the compound; contacting the sample with a noxious substance; and determining if the epithelia is damaged. These steps further the level of screening of the compound and therefore increase the likelihood of identification of an effective drug candidate. Techniques for performing the above-listed steps may be found in the art, particularly in: Tobey, N.A. *et al* (1986) *Am J Physiol* 251:G866-G869, Orlando, R.C. *et al* (1987) *Gastroenterology* 93:352-361, U.S. Patent No. 5,374,537, and U.S. Patent No. 5,189,056.

In particular embodiments, stratified squamous epithelial tissue may be obtained from esophageal, oral cavity, cheek, pharynx, larynx, or vocal cord tissue from an individual. A preferred embodiment is where the epithelia is obtained from rabbit esophageal tissue. Certain additional embodiments include tissue from rats, pigs, horses, or sheep and may also be selected from the forestomach or rumen of such animals. Additional embodiments include human epithelial tissue which may be obtained, for example from autopsy, surgical or biopsy specimens.

*In vivo,* epithelial tissue is exposed to gastrointestinal fluid, which comprises, in part, acid, acid-pepsin and other constituents, such as rennin, lipase, bile salts and acids, pancreatic enzymes such as trypsin, elastase, chymotrypsin and amylase, and hydrochloric acid (HCl). *In vitro* experiments performed on stratified squamous epithelial tissue in Ussing chambers, as discussed previously, for example in U.S. Patent Nos. 5,374,537 and 5,189, 056; Tobey, N.A. *et al* (1986) *Am J Physiol* 251:G866-G869; and Orlando, R.C. *et al* (1987) *Gastroenterology* 93:352-361 primarily use acid, acid-pepsin or N-acetylcysteine, a disulfide bond breaking molecule, to mimic the conditions existing *in vivo.*

Further embodiments of the above-described methods therefore include methods wherein the noxious substance may be N-acetylcysteine, acid, acid-pepsin or gastrointestinal fluid. In particular embodiments the noxious substance is gastric acid, such as HCl or HCl-pepsin. In other embodiments the noxious substance may comprise other acids such as phosphoric or sulfuric acid. Additional noxious substances include bile salts, bile acids, with and without HCl, nitric acid, acetic acid and other organic acids.

Particular embodiments of the invention may also include where the polypeptide is immobilized on a solid support. A variety of appropriate solid supports are known to persons of skill in the art. Exemplary supports may include beads, such as resin beads, slides, glass and plastic wells, test tubes, and plates. The attachment of the polypeptide to a solid support may facilitate performing the steps of the methods of the present invention, including, for example, contacting the polypeptide with a compound or noxious substance. Attachment to a solid support will also facilitate washing the polypeptide between steps. To a person of skill in the art, it will be apparent that any of the above described methods may comprise washing steps between, for example, the contact of the polypeptide with the compound and contact with a noxious substance. Further, washing after contacting the polypeptide with the noxious substance may also be performed before determining, for example, the degree of fragmentation or binding or stability of the polypeptide. The present invention also encompasses embodiments comprising at least one or multiple washing steps between at least one or more of the steps of the assay methods.

The following examples are meant by way of further description of the invention and are not intended to limit the invention described herein in any way.

### EXAMPLES

### EXAMPLE 1: Identification of the Protein Governing Paracellular Permeability in Rabbit Esophageal Epithelium

Sections of rabbit esophageal epithelium, mounted in Ussing chambers and stabilized for 45 min, were exposed both luminally and serosally to calcium-free solution containing EDTA, 3 mM. In the presence of a calcium-free solution there was a progressive decline in R, the decline shown for representative single tissues reaching 40% below the initial R at 1 hr, 60% at 2 hrs and 80% at 4 hrs. This contrasted with a control tissue in normal calcium-containing Ringer solution which showed a small increase in R of 10% over initial R at 1 hr, 12% at 2 hrs, and 12% at 4 hrs. The decline in R was the result of the calcium-free state and was not an effect of EDTA treatment since 3 mM EDTA alone had no effect on R over the described time frame.

When calcium-containing (normal Ringer) solution was restored to the bathing solutions after 1, 2, or 4 hrs of calcium-free exposure, there was a progressive return of R toward initial baseline values, with complete reversibility observed for tissue exposed to calcium-free solution for 1 hr and 2 hr exposures and an 80% recovery in R for tissue exposed to calcium-free for 4 hrs within 1 hr of restoring calcium to the baths. These results established that the protein(s) regulating paracellular permeability in the rabbit esophageal epithelium is calcium-dependent.

One protein known to be calcium-sensitive and to act as a bridge to seal off the intercellular space is the zonula adherens junctional protein, Uvomorulin, also known as E-cadherin (and also known as L-CAM, CAM 120/80, Arc-1). Gumbiner, *B.M et al* (1986) *J Cell Biol* 102:457-468.

E-cadherin is known to be insensitive to degradation by trypsin in the presence of calcium but sensitive to degradation in the absence of calcium (Takeichi, M. (1977) *J Cell Biol* 75:464-474). The junctional adhesive protein in esophageal epithelium was shown to be trypsin-sensitive in the absence, but not in the presence, of calcium, which further supports the conclusion that this adhesive protein is E-cadherin. Sections of rabbit esophageal epithelium were mounted in Ussing chambers and exposed luminally to trypsin, 0.01%, in the presence or absence of a calcium-free bathing solution. There was no significant change in R for tissues exposed to trypsin in the calcium-containing normal Ringer solution over a 2 hr period compared to tissues in calcium-containing normal Ringer not exposed to trypsin (R of 117±8% vs 107±8%, respectively, n=4, p>0.05). However, tissues exposed to calcium-free solution 3 mM EDTA both luminally and serosally with or without trypsin showed the expected decline in R over time, as evidenced by declines to 62±7% and 74±5%, respectively. Upon restoration of calcium to the baths, junctional resealing as evidenced by recovery in R, was shown to be inhibited at 1 hr in tissues exposed to trypsin compared to those unexposed to trypsin (62±8% vs 82±6%, respectively, n=4, p<0.05). This lack of recovery in R in tissues exposed to calcium-free solution suggests that trypsin degraded the junction adhesive protein, while the lack of trypsin's effect on R in calcium-containing solution suggests that the junctional protein is resistant to trypsin under these conditions.

Further, similar to the glycoprotein E-cadherin, whose sugar side chains are known to play little or no role in its ability to provide cell-to-cell adhesion (Takeichi, M. (1988) *Development* 102:639-655), the junctional protein that regulates paracellular permeability in esophageal epithelium was shown to be relatively insensitive to glycosidases. This was evident by the fact that luminal treatment of esophageal epithelium in Ussing Chambers with glycosidases had little (≤20% decrease) or no (decrease approximately 0%) effect on junctional permeability as reflected by change in R.

E-cadherin contains cysteine residues with disulfide bridges in its extracellular domain (Takeichi, M. (1988) *Development* 102:639-655). The junctional protein regulating paracellular permeability in esophageal epithelium is also highly sensitive to disulfide-bond breaking compounds such as N-acetylcysteine. U.S. Pat. No. 5,374,537; 5,189,056. Further, like E-cadherin, which is known to be heat labile, the junctional protein regulating paracellular permeability in esophageal epithelium is heat-sensitive, *i*.*e*. heating the luminal bath resulted in a temperature-sensitive decline in R (Tobey, N.A. *et al* (1999) *Am J Physiol* 276:G1322-G1330).

### EXAMPLE 2: Junctional Resealing In Esophageal Epithelium is Inhibited by Exposure to Mab to the Extracellular Domain Of E-Cadherin

Sections of rabbit esophageal epithelium prepared as in Example 1 were exposed luminally and serosally to a calcium-free solution containing 3 mM EDTA to open the junctions as shown by the progressive decline in R to 60±4% and 65±4% of initial R for experimental and control tissues, respectively, by 2 hrs (n = 8 for each group, p > 0.05). Tissues were then treated luminally for 20 min with either 10 µg/ml of a monoclonal antibody (Mab) to Uvomorulin/E-cadherin (Sigma Chemical, St. Louis, MO) or, as control, to 10 µg/ml of nonspecific immune serum globulin (IgG). Following exposure to Mab or IgG, a calcium-containing (normal Ringer) solution was restored to the bathing solutions and the recovery of R monitored over time. Tissues treated with the Mab to Uvomorulin/E-cadherin showed significant inhibition in recovery of R toward baseline compared to that of tissues treated with IgG; recovery being 70±5% for Mab vs 86±6% for IgG by 1 hr after calcium restoration and 73±4% for Mab vs 88±4% for IgG by 2 hrs (p< 0.05 for each comparison). This indicated that the resealing of the junctions in stratified squamous epithelium is dependent upon the adhesive function of Uvomorulin/E-cadherin.

The intercellular junctions of stratified squamous epithelium can also be opened by exposure to hypertonic saline. Junctional opening was indicated by a drop in R. Treatment with the same Mab to Uvomorulin/E-cadherin as above also blocked the resealing of the junctions, *i*.*e*. return of R to baseline, upon reestablishing the isoosmolality of the bathing solution.

An antibody to the *intracellular* domain of a cadherin (N-cadherin) was also similarly tested and found to have no ability to block junctional resealing, i.e. tissues exposed to Mab to the intracellular domain of N-cadherin recovered to 104%±5%, using IgG recovery as 100% as control (n=3, p>0.05). This supports the specificity for inhibition of junctional resealing by Mab to the *extracellular* domain of Uvomorulin/E-cadherin.

### EXAMPLE 3: Junctional Resealing in Esophageal Epithelium is not Inhibited by Mabs to Occludin or Claudin-1

Sections of rabbit esophageal epithelium prepared as in Example 1 were exposed luminally and serosally to a calcium-free solution containing 3 mM EDTA to open the junctions as shown by the progressive decline in R over 2 hrs to approximately 40% of initial R. Tissues were then treated luminally for 20 min with either 10 µg/ml of a monoclonal antibody (Mab) to Occludin or a polyclonal antibody to Claudin-1 (both from Zymed, San Francisco, CA), or to nonspecific immune serum globulin (IgG), as control. Following the restoration of calcium-containing (normal Ringer) solution to the bathing solutions, the recovery of R toward baseline was monitored. Unlike the prior inhibition noted with Mab to Uvomorulin/E-cadherin, R had increased similarly by 2 hrs after calcium replacement for tissues exposed to antibodies to occludin or claudin-1 compared with the IgG controls. For example, at 2 hrs post-calcium restoration, using IgG recovery as 100% as control, tissues exposed to Mab for occludin recovered to 96%±6%, and to polyclonal antibodies to claudin-1 recovered to 108%+8% (n=3 per group, p> 0.05 compared to control IgG for each group). This demonstrated that junctional resealing in esophageal epithelium was independent of the adhesive function of occludin or claudin-1, junctional proteins which are not members of the cadherin superfamily of proteins.

### EXAMPLE 4: Junctional Resealing in Esophageal Epithelium Inhibited by Mab to Desmogleins 1 and 2 and Desmocollin 3

Sections of rabbit esophageal epithelium prepared as in Example 1 were exposed luminally and serosally to calcium-free solution containing 3 mM EDTA to open the junctions as shown by the progressive decline in R over 2 hrs to approximately 40% of initial R. Tissues were then treated luminally for 20 min with either 10 µg/ml of a Mab to desmoglein 1 and 2 (Research Diagnostics, Flanders, NJ), desmocollin 3 (Research Diagnostics, Flanders, NJ), Uvomorulin/E-cadherin (Sigma Chemical, St. Louis, MO), or nonspecific immune serum globulin (IgG) as control. Following restoration of calcium-containing (normal Ringer) solution, the Mab for desmoglein, desmocollin and Uvomorulin/E-cadherin were observed to significantly inhibit return of R toward initial values compared to controls exposed to IgG. For example, when expressed as a percent recovery in relation to IgG (IgG recovery normalized to 100%) at 2 hrs, Uvomorulin Mab was 83±6%, desmocollin Mab was 83±0.5% and desmoglein 1 and 2 Mab was 90±8% (n=4-6/group, p<0.05 for each group compared to the IgG control). This indicated that junctional resealing in esophageal epithelium can also be inhibited by Mab to desmoglein 1 and 2, and desmocollin 3, which are members of the cadherin superfamily of proteins with known sequence homology to Uvomorulin/E-cadherin.

The ability of desmosomal antibodies for desmoglein 1 and 2 or desmocollin 3 to block resealing similar to that of Mab to Uvomorulin/E-cadherin is likely due to homology between the proteins desmoglein 1 and 2, desmocollin 3 and Uvomorulin/E-eadherin. Desmosomes are structures that provide tensile strength to the epithelium but do not regulate permeability through the paracellular pathway, and thus the mechanism of action is not by Mab binding to desmogleins 1 or 2, or desmocollin 3. (Gumbiner, B.M. (1996) *Cell* 84:345-357, Swami, S. *et al* (1995) *Am J Gastroenterol* 90:1808-1813, Dobson, H. *et al* (1994) *Gut* 35:1343-1347).

### EXAMPLE 5: Method for Measuring Mab Competition with Mab to E-cadherin for Binding to Extracellular Domain of E-cadherin

A commercial immunoassay test kit (Human E-cadherin EIA kit, Zymed Laboratories, San Francisco, CA) that contains an antigen (Uvomorulin/E-cadherin), and a Mab to the extracellular domain of Uvomorulin/E-cadherin was used to demonstrate that Mab to desmocollin 3 can bind to the extracellular domain of Uvomorulin/E-cadherin.

The kit enables the antigen to be exposed to agents or antibodies prior to exposure to the standard Mab to the extracellular domain of Uvomorulin/E-cadherin. The ability of the agent or antibody to block the binding of the Mab to the extracellular domain of Uvomorulin/E-cadherin can then be used to determine if competition for binding sites on the antigen has occurred. Comparative binding is based on the quantity of binding observed when the Mab to Uvomorulin/E-cadherin is placed in contact with antigen in the absence of prior exposure to test agent or antibody. The experimental control for test agents is vehicle (normal Ringer solution) and for monoclonal or polyclonal antibodies is an equivalent amount of nonspecific immune serum globulin (IgG). The quantity of binding of the Mab to Uvomorulin/E-cadherin to antigen in the absence of test agent or test antibody is labeled as control and whose value is normalized to 100%.

Antigen which was first exposed to a similar quantity of a Mab to desmocollin 3 (3 µg/100 µL) (Research Diagnostics, Flanders, NJ) prior to exposure of the Mab to Uvomorulin/E-cadherin demonstrated significantly reduced binding of Uvomorulin/E-cadherin Mab (64±5.8% compared to 3 µg/100 µL of IgG as control, p<0.05, n=6). This indicated that the Mab to desmocollin 3 competes effectively for the same binding sites as the Mab to the extracellular domain of Uvomorulin/E-cadherin.

Without being bound to any particular theory, it appears, in effect, that the Mab to desmoglein 1 and 2, and desmocollin 3 exert their effect on resealing by binding to the extracellular domain of Uvomorulin/E-cadherin. This conclusion is further supported by the fact that the Mab to desmoglein 1 and 2, and desmocollin 3 cross react on SDS gel electrophoresis with one or more of the same protein bands as the Mab to Uvomorulin/E-cadherin.

### EXAMPLE 6: SITS Provides Protection Against Noxious Substances

Uvomorulin/E-cadherin was identified by SDS gel electrophoresis in homogenates of esophageal epithelium. These findings are supported by reports from others of the presence of Uvomorulin/E-cadherin on immunostaining, and on SDS gel electrophoresis in human esophageal epithelium (Swami, S. *et al* (1995) *Am J Gastroenterol* 90:1808-1813).

When epithelia were treated with acid-pepsin or N-acetylcysteine and then homogenized, bands which stain with the Mab to E-cadherin are visible at 50kD and 80kD. The band at approximately 80 kD should correspond to the extracellular domain of E-cadherin, as identified elsewhere, after treatment of E-cadherin with trypsin in the absence of calcium, an approximately 80kD band is identified. Gumbiner, B.M. *et al.* (1986) *J Cell Biol* 102:457-468.

SITS was selected to investigate the mechanism of protection because of its commercial availability, high level of protection against acid injury to esophageal epithelium, and lack of reversibility of its protective effect even with repeated washes of the tissue prior to acid exposure. When epithelia were treated first with SITS, and then contacted with acid-pepsin or N-acetylcysteine, the two bands at approximately 50 kD were not seen, suggesting that the cytoprotective agent SITS prevents the breakdown or fragmentation of the extracellular domain in the presence of the noxious substances acid-pepsin and N-acetylcysteine.

Protection of Uvomorulin/E-cadherin against damage by acid or N-acetylcysteine was shown to be direct, *i.e*., by binding of the compound to the protein itself (rather than indirect by the binding of the compound to other proteins providing a shield against the access of acid or N-acetylcysteine to the critical junctional protein). Sections of rabbit esophageal epithelium were mounted in Ussing chambers and exposed luminally to either SITS, 4 mM, or vehicle (Ringer) as control for 30 min. Following exposures, both bathing solutions were made calcium-free solutions (3 mM EDTA and 4 mM SITS, or 3 mM EDTA without SITS (control)) to open the junctions as evidenced by the decline in R. In the presence of calcium-free solution with or without 4 mM SITS, R over a 2 hr period declined similarly for the two groups to 68±3% or 67+3 %, respectively. However, recovery of R following restoration of calcium-containing bathing solutions (0 mM SITS) showed that the recovery of R of tissues previously exposed to SITS was significantly reduced over that of the unexposed tissue (SITS treated recovery of 75±3% vs untreated controls of 90±5%, n=3, p<0.05). This showed that luminal SITS inhibits junctional resealing, a function of the adhesive protein E-cadherin, and this interference is likely through its ability to bind to the extracellular domain of E-cadherin.

### EXAMPLE 7: Method for Determining the Ability of Compounds to Bind to the Extracellular Domain of Uvomorulin/E-Cadherin

The Human E-cadherin EIA kit (Zymed Laboratories, San Francisco, CA) was used as in the method described in Example 5 to determine binding of test compounds, SITS, SOS, CDDD-1192, CDDD-1193 and 1,3-benzene disulfonic acid (1,3-BDSA) to the approximately 80 kD extracellular domain of Uvomorulin/E-Cadherin provided in the E-cadherin EIA kit. This was done by adding the extracellular domain of human Uvomorulin/E-Cadherin to a solution with varying concentrations of the compounds in the nanomolar to millimolar range or with vehicle (control, normal Ringer solution) for 30 min. Following incubation for 2 hrs, the solution is added to a solid phase microtiter plate, provided in the EIA kit, to which a mouse monoclonal anti-human Uvomorulin/E-cadherin antibody (Zymed Laboratories, San Francisco, CA) is bound. This permits antigen with epitopes unoccupied by test compound to bind to the plate. After washing the plate with Tris buffer containing 0.1 % Tween-20 (Promega, Madison, Wisconsin) and 5 mM calcium chloride, the second mouse monoclonal anti-human Uvomorulin/E-cadherin antibody labeled with peroxidase is used to tag the bound antigen. After an additional incubation for 1 hr and washing with Tris buffer, the peroxidase is detected with substrate (hydrogen peroxide and tetramethyl-benzidine in buffered solution, as included in the EIA test kit) by incubating for 15 min at room temperature and stopping of the reaction with 1N H₂O₄. This results in color development with intensities proportional to the amount of Uvomorulin/E-cadherin present in the samples. The amount of Uvomorulin/E-cadherin is quantitated by measuring absorbance at 450 nm within an hour following development using an EIA plate reader (Vmax Plate Reader, Molecular Devices, Sunnyvale, CA). The degree of binding of a compound to the extracellular domain of Uvomorulin/E-cadherin is determined by the reduction in antigen detection in the plate exposed to test compound compared to that for the control plate (100%) where no compound was present (Table 2).

**Table 2.**

| The ability of selected agents to alter binding of a Mab to the extracellular domain of Uvomorulin/E-cadherin to its antigenic sites. | | |
|---|---|---|
| Agent | Concentration | % Binding |
| Control (vehicle) | | 100 |
| 1,3 BDSA | 4 mM | 104±3 |
| SITS | 40 uM | 77±3* |
| SITS | 4 mM | 26±3* |
| SITS | 8 mM | 20±3* |
| SOS | 4 mM | 83±0.3* |
| SOS | 10 mM | 76±0.3* |
| CDDD-1192 | 0.4 uM | 103±6 |
| CDDD-1192 | 4 mM | 77±13* |
| CDDD-1193 | 40 nM | 110±6 |
| CDDD-1193 | 40 uM | 83±3* |
| CDDD-1193 | 4 mM | 38±0.75* |

| | | |
|---|---|---|
| * p <0.05 compared to control; n=3-6 for each experimental group | | |

The experimental results depicted in Table 2 establish the ability of protective doses of SITS, SOS, CDDD-1192 and CDDD-1193, but not non-protective doses of 1,3-BDSA, a compound ineffective against acid injury in the Ussing chamber experiments at a concentration of 4 mM (as shown in U.S. Patent No. 5,189,056 and 5,374,537), to bind to the extracellular domain of E-cadherin. Further, the binding affinity for the epitope identified by the Mab to the extracellular domain for E-cadherin is greatest for CDDD-1193 and SITS at 4 mM concentrations when compared to that of CDDD-1192 or SOS.

Esophageal epithelium luminally pretreated with SITS (even without prior junctional opening by calcium-free solution) is found to inhibit the binding of a Mab to Uvomorulin/E-cadherin to its antigen (Uvomorulin/E-cadherin) when applied to homogenized epithelium run on SDS gel electrophoresis.

Additionally, esophageal epithelium luminally pretreated with SITS in Ussing chambers that were subsequently exposed to acid-pepsin or N-acetylcysteine were resistant to the degradation of Uvomorulin/E-cadherin into component proteins (Example 6). This was demonstrated by showing additional protein bands with Mab staining for Uvomorulin/E-cadherin of homogenized esophageal epithelium on SDS gel electrophoresis for tissues exposed to acid-pepsin or N-acetylcysteine compared to similarly exposed tissues that were pretreated with luminal SITS.

Taken together, these data support the conclusion that the previously identified luminally-active cytoprotective compounds inhibit the junctional breakdown (and concomitant increase in paracellular permeability) in esophageal epithelium upon direct damage by acid, acid-pepsin or N-acetylcysteine, by direct binding to the extracellular domain of Uvomorulin/E-cadherin. Without being bound by any particular theory, it appears that the mechanism of action of these compounds is the stabilization of the target protein, *i*.*e*. Uvomorulin/E-cadherin, which results in protection against acid, acid-pepsin or N-acetylcysteine induced increases in junctional (paracellular) permeability.

It appears that sulfonates like SITS and sulfate esters, e.g. SOS, CDDD-1192 and CDDD-1193, act via the same mechanism. Sucrose octasulfate (previously shown to irreversibly protect against acid-induced injury to esophageal epithelium (U.S. Patent No. 5,374,537 and 5,189,056)) also dose-dependently binds to the extracellular domain of Uvomorulin/E-cadherin.

A third category of alleged cytoprotective compounds known as inorganic tetrahedral-shaped oxyanions containing compounds such as sulfates, molybdates, chromates, and tungstates did not, like organic sulfonates and sulfate esters, inhibit binding of the Mab to the extracellular domain of Uvomorulin/E-cadherin to its antigen, Uvomorulin/E-cadherin, using a commercially available immunoassay kit, as described above. This likely reflects the fact that these compounds demonstrate readily reversible protection against acid injury in the Ussing chamber, such that their binding to the protective site is most likely reversible and so ineffective at inhibiting the Mab to Uvomorulin/E-cadherin from binding to its antigen. Thus, compounds like the inorganic tetrahedral-shaped oxyanions described may show cytoprotective activity in rabbit esophageal epithelial tissue models as described in U.S. Pat. Nos. 5,374,537 and 5,189,056, but may not be suitable for use as control cytoprotective agents in the assay methods described herein. Further, the reversibility of binding suggests that these compounds may also not be as effective *in vivo,* where the esophageal tissues are being washed by saliva.

Identification of the isolated extracellular domain of Uvomorulin/E-cadherin as the protective site supports use of this protein and its extracellular domain specifically as a test system for identifying, screening, selecting and designing compounds, chemicals, molecules, antibodies or other substances for protective effects against luminal damaging compounds, e.g. HCI, to stratified squamous epithelia.

Modifications of the above described modes for carrying out the invention that are obvious to those of ordinary skill in the chemical, pharmaceutical, or related arts are intended to be within the scope of the following claims.

## Claims

1. A method of assaying for a compound which may protect stratified squamous epithelia from damage by a noxious substance, comprising in order:
a) contacting a polypeptide with a compound; wherein the polypeptide is selected from the group consisting of an extracellular portion of a cadherin or a peptide sequence having at least 13% sequence identity to a sequence extending from amino acid residue 155 through amino acid residue 262 of SEQ ID NO:1; and
b) determining whether the compound binds to the polypeptide;
wherein binding of the compound to the polypeptide indicates that the compound may protect stratified squamous epithelia from damage by a noxious substance.

2. The method of claim 1, further comprising:
c) comparing the binding of the compound to the polypeptide with the binding of a cytoprotective agent to the polypeptide;
wherein a greater than or equal level of binding by the compound indicates that the compound may protect stratified squamous epithelia from damage by the noxious substance.

3. The method of claim 1, wherein the polypeptide comprises the extracellular domain 1 of a cadherin superfamily member selected from the group consisting of E-cadherin, desmocollin 3, desmoglien 1, desmoglien 2 and desmoglien 3.

4. The method of claim 3, wherein the polypeptide comprises the extracellular domain 1 of E-cadherin.

5. The method of claim 1, wherein the polypeptide comprises a sequence selected from the group consisting of SEQ ID NOS:6-21, and SEQ ID NOS:22-25.

6. The method of claim 1, wherein the polypeptide comprises one or more sequences selected from the group consisting of residues 155-262 of SEQ ID NO:1, residues 52-157 of SEQ ID NO:2, residues 49-159 of SEQ ID NO:3, residues 52-157 of SEQ ID NO:4, and residues 136-243 of SEQ ID NO:5.

7. The method of claim 1, further comprising in order:
c) contacting a sample comprising stratified squamous epithelial tissue with the compound;
d) contacting the sample with a noxious substance; and
e) determining if the epithelia is damaged.

8. The method of claim 7, wherein the noxious substance is selected from the group consisting of gastric acid, HCl, N-acetylcysteine, pepsin, acid-pepsin, and gastrointestinal fluid.

9. The method of claim 1, further comprising:
before step a), contacting the polypeptide with a labelled cytoprotective agent; and
after step b), comparing binding of the agent to the polypeptide contacted with the compound to a control conducted where the polypeptide is contacted with the agent and is not contacted with the compound;
wherein a decrease in binding of the agent to the polypeptide contacted with the compound compared to the control indicates that the compound may protect stratified squamous epithelia from damage by a noxious substance.

10. The method of claim 9, wherein the agent is labelled with a radioactive isotope, fluorescent label or electron dense tracer.

11. The method of claim 1, further comprising:
after step a) and before step b), contacting the polypeptide with a monoclonal antibody to the polypeptide; and
after step b), comparing the amount of antibody binding to the polypeptide contacted with the compound to the amount of antibody binding to a control;
wherein a decrease in the amount of antibody binding to the polypeptide which has been contacted with the compound compared to the control indicates that the compound may protect stratified squamous epithelia from damage by a noxious substance.

12. A method of assaying for a compound which may protect stratified squamous epithelia from damage by a noxious substance, comprising in order:
a) contacting a polypeptide with a compound; wherein the polypeptide is selected from the group consisting of an extracellular portion of a cadherin or a peptide sequence having at least 13% sequence identity to a sequence extending from amino acid residue 155 through amino acid residue 262 of SEQ ID NO:1; and
b) determining the stability of the polypeptide; and
c) comparing the stability of the polypeptide contacted with the compound to the stability of a control;
wherein a greater stability for the polypeptide contacted with the compound compared to the control indicates that the compound may protect stratified squamous epithelia from damage by a noxious substance.

13. The method of claim 12, further comprising:
after step a) and before step b), contacting the polypeptide with a noxious substance;
such that step c) compares the stability of the polypeptide contacted with the compound and the noxious substance;
wherein if the stability of the polypeptide contacted with the compound and noxious substance is greater than that of the control, the compound may protect stratified squamous epithelium against damage by noxious substance.

14. The method of claim 13, wherein the measure of stability of the peptide is the amount of fragmentation of the polypeptide, and wherein a decrease in the amount of fragmentation of the polypeptide contacted with the compound compared to the control indicates that the compound may protect stratified squamous epithelia from damage by a noxious substance.

15. The method of claim 14, wherein fragmentation is determined using a technique from the group consisting of SDS gel electrophoresis, mass spectrometry, HPLC, or amino acid analysis.

## Patentansprüche

1. Verfahren zum Testen auf eine Verbindung, die geschichtete Schuppenepithele vor Schädigungen durch eine schädliche Substanz schützen kann, umfassend in nachstehender Reihenfolge:
a) Kontaktieren eines Polypeptids mit einer Verbindung, worin das Polypeptid aus der aus einem extrazellulären Abschnitt eines Cadherins oder einer Peptidsequenz mit zumindest 13 % Sequenzidentität mit einer Sequenz, die sich von Aminosäurerest 155 bis zu Aminosäurerest 262 aus Seq.-ID Nr. 1 erstreckt, bestehenden Gruppe ausgewählt ist; und
b) Bestimmen, ob sich die Verbindung an das Polypeptid bindet; worin das Binden der Verbindung an das Polypeptid darauf hinweist, dass die Verbindung geschichtete Schuppenepithele vor Schädigungen durch eine schädliche Substanz schützen kann.

2. Verfahren nach Anspruch 1, weiters umfassend:
c) Vergleichen der Bindung der Verbindung an das Polypeptid mit der Bindung eines zellschützenden Mittels an das Polypeptid;
worin ein höheres oder gleiches Bindungsniveau durch die Verbindung darauf hinweist, dass die Verbindung geschichtete Schuppenepithele vor Schädigungen durch eine schädliche Substanz schützen kann.

3. Verfahren nach Anspruch 1, worin das Polypeptid die extrazelluläre Domäne 1 eines Mitglieds der Cadherin-Überfamilie, ausgewählt aus der aus E-Cadherin, Desmocollin 3, Desmoglien 1, Desmoglien 2 und Desmoglien 3 bestehenden Gruppe, umfasst.

4. Verfahren nach Anspruch 3, worin das Polypeptid die extrazelluläre Domäne 1 von E-Cadherin umfasst.

5. Verfahren nach Anspruch 1, worin das Polypeptid eine Sequenz, ausgewählt aus der aus Seq.-ID Nr. 6-21 und Seq.-ID Nr. 22-25 bestehenden Gruppe, umfasst.

6. Verfahren nach Anspruch 1, worin das Polypeptid eine oder mehrere Sequenzen, ausgewählt aus der aus den Resten 155-262 von Seq.-ID Nr. 1, den Resten 52-157 aus Seq.-ID Nr. 2, den Resten 49-159 aus Seq.-ID Nr. 3, den Resten 52-157 aus Seq.-ID Nr. 4 und den Resten 136-243 aus Seq.-ID Nr. 5 bestehenden Gruppe, umfasst.

7. Verfahren nach Anspruch 1, weiters in der folgenden Reihenfolge umfassend:
c) Kontaktieren einer Probe, die geschichtetes Schuppenepithelgewebe umfasst, mit der Verbindung;
d) Kontaktieren der Probe mit einer schädlichen Substanz; und
e) Bestimmen, ob die Epithele geschädigt sind.

8. Verfahren nach Anspruch 7, worin die schädliche Substanz aus der aus Magensäure, HCl, N-Acetylcystein, Pepsin, Säurepepsin und Gastrointestinalflüssigkeit bestehenden Gruppe ausgewählt ist.

9. Verfahren nach Anspruch 1, weiters umfassend:
vor Schritt a) Kontaktieren des Polypeptids mit einem markierten zellschützenden Mittel; und
nach Schritt b) Vergleichen der Bindung des Mittels an das Polypeptid, das mit der Verbindung kontaktiert wurde, mit einer Kontrolle, die durchgeführt wurde, worin das Polypeptid mit dem Mittel kontaktiert wurde, jedoch nicht mit der Verbindung;
worin ein Rückgang der Bindung des Mittels an das Polypeptid, das mit der Verbindung kontaktiert wurde, im Vergleich zur Kontrolle darauf hinweist, dass die Verbindung geschichtete Schuppenepithele vor Schädigungen durch eine schädliche Substanz schützen kann.

10. Verfahren nach Anspruch 9, worin das Mittel mit einem radioaktiven Isotop, einer fluoreszierenden Markierung oder einem Elektronendichteindikator markiert ist.

11. Verfahren nach Anspruch 1, weiters umfassend:
nach Schritt a) und vor Schritt b) das Kontaktieren des Polypeptids mit einem gegen das Polypeptid gerichteten monoklonalen Antikörper; und
nach Schritt b) das Vergleichen der Menge an Antikörpern, die sich an das mit der Verbindung kontaktierte Polypeptid binden, mit der Menge an Antikörpern, die sich an eine Kontrolle binden;
worin ein Rückgang der Menge an Antikörpern, die sich an das Polypeptid binden, das mit der Verbindung kontaktiert wurde, im Vergleich mit der Kontrolle darauf hinweist, dass die Verbindung geschichtete Schuppenepithele vor Schädigungen durch eine schädliche Substanz schützen kann.

12. Verfahren zum Testen auf eine Verbindung, die geschichtete Schuppenepithele vor Schädigungen durch eine schädliche Substanz schützen kann, umfassend in nachstehender Reihenfolge:
a) Kontaktieren eines Polypeptids mit einer Verbindung, worin das Polypeptid aus der aus einem extrazellulären Abschnitt eines Cadherins oder einer Peptidsequenz mit zumindest 13 % Sequenzidentität mit einer Sequenz, die sich von Aminosäurerest 155 bis zu Aminosäurerest 262 aus Seq.-ID Nr. 1 erstreckt, bestehenden Gruppe ausgewählt ist; und
b) Bestimmen der Stabilität des Polypeptids; und
c) Vergleichen der Stabilität des Polypeptids, das mit der Verbindung kontaktiert wurde, mit der Stabilität einer Kontrolle;
worin eine größere Stabilität des Polypeptids, das mit der Verbindung kontaktiert wurde, im Vergleich mit der Kontrolle darauf hinweist, dass die Verbindung geschichtete Schuppenepithele vor Schädigungen durch eine schädliche Substanz schützen kann.

13. Verfahren nach Anspruch 12, weiters umfassend:
nach Schritt a) und vor Schritt b) das Kontaktieren des Polypeptids mit einer schädlichen Substanz;
sodass Schritt c) die Stabilität des Polypeptids, das mit der Verbindung und der schädlichen Substanz kontaktiert wurde, vergleicht;
worin, sofern die Stabilität des Polypeptids, das mit der Verbindung und der schädlichen Substanz kontaktiert wurde, größer als jene der Kontrolle ist, die Verbindung geschichtete Schuppenepithele vor Schädigungen durch eine schädliche Substanz schützen kann.

14. Verfahren nach Anspruch 13, worin das Maß für die Stabilität des Peptids die Menge an Fragmentierung des Polypeptids ist und worin ein Rückgang der Menge an Fragmentierungen des Polypeptids, das mit der Verbindung kontaktiert wurde, verglichen mit der Kontrolle darauf hinweist, dass die Verbindung geschichtete Schuppenepithele vor Schädigungen durch eine schädliche Substanz schützen kann.

15. Verfahren nach Anspruch 14, worin Fragmentierung unter Verwendung eines aus der aus SDS-Gel-Elektrophorese, Massenspektrometrie, HPLC oder Aminosäureanalyse bestehenden Gruppe ausgewählten Verfahrens bestimmt wird.

## Revendications

1. Un procédé d'analyse d'un composé susceptible de protéger les épithéliums pavimenteux stratifiés des dommages causés par une substance nocive, comprenant dans l'ordre:
a) la mise en contact d'un polypeptide avec un composé; où le polypeptide est choisi dans le groupe se composant d'une partie extracellulaire d'une cadhérine ou d'une séquence peptidique ayant au moins 13% d'identité de séquence à une séquence s'étendant du résidu d'acide aminé 155 au résidu d'acide aminé 262 de ID SEQ NO:1; et
b) la détermination que le composé se lie ou non au polypeptide;
où la liaison du composé au polypeptide indique que le composé est susceptible de protéger les épithéliums pavimenteux stratifiés des dommages causés par une substance nocive.

2. Le procédé de la revendication 1, comprenant en outre:
c) la comparaison de la liaison du composé au polypeptide avec la liaison d'un agent cytoprotecteur au polypeptide;
où un niveau de liaison supérieur ou égal par le composé indique que le composé est susceptible de protéger les épithéliums pavimenteux stratifiés des dommages causés par la substance nocive.

3. Le procédé de la revendication 1, dans lequel le polypeptide comprend le domaine extra -cellulaire 1 d'un élément de la superfamille de la cadhérine choisi dans le groupe se composant de : E -cadhérine, desmocolline 3, desmoglien 1, desmoglien 2 et desmoglien 3.

4. Le procédé de la revendication 3, dans lequel le polypeptide comprend le domaine extracellulaire 1 de E - cadhérine.

5. Le procédé de la revendication 1, dans lequel le polypeptide comprend une séquence choisie dans le groupe se composant de ID SEQ NO:6 -21 et ID SEQ NO:22 - 25.

6. Le procédé de la revendication 1, dans lequel le polypeptide comprend une ou plusieurs séquences choisies dans le groupe se composant de résidus 155-262 de ID SEQ NO:1, résidus 52-157 de ID SEQ NO:2, résidus 49-159 de ID SEQ NO:3, résidus 52-157 de ID SEQ NO:4 et résidus 136-243 de ID SEQ NO:5.

7. Le procédé de la revendication 1, comprenant en outre, dans l'ordre;
c) la mise en contact d'un échantillon comprenant un tissu épithélial pavimenteux stratifié avec le composé;
d) la mise en contact de l'échantillon avec une substance nocive; et
e) la déterminaion que les épithéliums sont ou non endommagés.

8. Le procédé de la revendication 7, dans lequel la substance nocive est choisie dans le groupe se composant de l'acide gastrique, de l'HCl, de la N-acétylcystéine, de la pepsine, de l'acide-pepsine et du fluide gastro-intestinal.

9. Le procédé de la revendication 1, comprenant en outre:
avant l'étape a), la mise en contact du polypeptide avec un agent cytoprotecteur marqué; et
après l'étape b), la comparaison de la liaison de l'agent au polypeptide mis en contact avec le composé à un témoin réalisé là où le polypeptide est mis en contact avec l'agent et n'est pas mis en contact avec le composé.
où une diminution dans la liaison de l'agent au polypeptide mis en contact avec le composé par rapport au témoin indique que le composé est susceptible de protéger les épithéliums pavimenteux stratifiés des dommages causés par une substance nocive.

10. Le procédé de la revendication 9, dans lequel l'agent est marqué à l'aide d'un isotope radioactif, d'un marqueur fluorescent ou d'un traceur opaque aux électrons.

11. Le procédé de la revendication 1, comprenant en outre:
après l'étape a) et avant l'étape b), la mise en contact du polypeptide avec un anticorps monoclonal au polypeptide; et
après l'étape b), la comparaison de la quantité d'anticorps se liant au polypeptide mis en contact avec le composé à la quantité d'anticorps se liant à un témoin;
où une diminution dans la quantité d'anticorps se liant au polypeptide qui a été mis en contact avec le composé par rapport au témoin indique que le composé est susceptible de protéger des épithéliums pavimenteux stratifiés des dommages causés par une substance nocive.

12. Un procédé d'analyse d'un composé susceptible de protéger les épithéliums pavimenteux stratifiés des dommages causés par une substance nocive, comprenant dans l'ordre:
a) la mise en contact d'un polypeptide avec un composé; où le polypeptide est choisi dans le groupe se composant d'une partie extracellulaire d'une cadhérine ou d'une séquence peptidique ayant au moins 13% d'identité de séquence à une séquence s'étendant du résidu d'acide aminé 155 au résidu d'acide aminé 262 de ID SEQ NO:1; et
b) la détermination de la stabilité du polypeptide; et
c) la comparaison de la stabilité du polypeptide mis en contact avec le composé à la stabilité d'un témoin;
où une plus grande stabilité pour le polypeptide mis en contact avec le composé par rapport au témoin indique que le composé est susceptible de protéger les épithéliums pavimenteux stratifiés des dommages causés par une substance nocive.

13. Le procédé de la revendication 12, comprenant en outre:
après l'étape a) et avant l'étape b), la mise en contact du polypeptide avec une substance nocive;
telle que l'étape c) compare la stabilité du polypeptide mis en contact avec le composé et la substance nocive;
où si la stabilité du polypeptide mis en contact avec le composé et la substance nocive est supérieure à celle du témoin, le composé est susceptible de protéger l'épithélium pavimenteux stratifié contre les dommages causés par une substance nocive.

14. Le procédé de la revendication 13, dans lequel la mesure de la stabilité du polypeptide est la quantité de fragmentation du polypeptide, et dans lequel une diminution dans la quantité de fragmentation du polypeptide mis en contact avec le composé par rapport au témoin indique que le composé est susceptible de protéger les épithéliums pavimenteux stratifiés des dommages causés par une substance nocive.

15. Le procédé de la revendication 14, dans lequel la fragmentation est déterminée en utilisant une technique comprise dans le groupe se composant de : électrophorèse sur gel de dodécylsulfate de sodium, spectrométrie de masse, chromatographie liquide à haute pression, ou analyse d'acides aminés.


### SEQUENCE LISTING

<110> The Administrators of the Tulane Educational Fund Tobey, Nelia A. Orlando, Roy C.
<120> Assay Methods for Identifying Compounds Which May Protect Stratified Squamous Epithelium Against Damage By Noxious Substances
<130> 484482000240
<140> To Be Assigned
   <141> Herewith
<150> 09/626,196
   <151> 2000-07-28
<160> 25
<170> FastSEQ for Windows Version 4.0
<210> 1
   <211> 882
   <212> PRT
   <213 > Homo Sapien
<220>
   <221> REPEAT
   <222> (155) ... (262)
   <223> Extracellular subdomain 1
<221> REPEAT
   <222> (263) ... (375)
   <223> Extracellular subdomain 2
<221> REPEAT
   <222> (376) ... (486)
   <223> Extracellular subdomain 3
<221> REPEAT
   <222> (487) ... (593)
   <223> Extracellular subdomain 4
<221> REPEAT
   <222> (594) ... (697)
   <223> Extracellular subdomain 5
<221> DOMAIN
   <222> (155) ... (707)
   <223> Extracellular domain
<400> 1
<210> 2
   <211> 1049
   <212> PRT
   <213> Homo Sapien
<220>
   <221> DOMAIN
   <222> (50)...(548)
   <223> Extracellular domain
<400> 2
   Met Asp Trp Ser Phe Phe Arg Val Val Ala Val Leu Phe Ile Phe Leu
    1               5                  10                  15
   Val Val Val Glu Val Asn Ser Glu Phe Arg Ile Gln Val Arg Asp Tyr
               20                  25                  30
   Asn Thr Lys Asn Gly Thr Ile Lys Trp His Ser Ile Arg Arg Gln Lys
   35 40 45
   Arg Glu Trp Ile Lys Phe Ala Ala Ala Cys Arg Glu Gly Glu Asp Asn
   50 55 60
   Ser Lys Arg Asn Pro Ile Ala Lys Ile His Ser Asp Cys Ala Ala Asn
   65 70 75 80
   Gln Gln Val Thr Tyr Arg Ile Ser Gly Val Gly Ile Asp Gln Pro Pro
   85 90 95
   Tyr Gly Ile Phe Val Ile Asn Gln Lys Thr Gly Glu Ile Asn Ile Thr
   100 105 110
   Ser Ile Val Asp Arg Glu Val Thr Pro Phe Phe Ile Ile Tyr Cys Arg
   115 120 125
   Ala Leu Asn Ser Met Gly Gln Asp Leu Glu Arg Pro Leu Glu Leu Arg
   130 135 140
   Val Arg Val Leu Asp Ile Asn Asp Asn Pro Pro Val Phe Ser Met Ala
   145 150 155 160
   Thr Phe Ala Gly Gln Ile Glu Glu Asn Ser Asn Ala Asn Thr Leu Val
   165 170 175
   Met Ile Leu Asn Ala Thr Asp Ala Asp Glu Pro Asn Asn Leu Asn Ser
   180 185 190
   Lys Ile Ala Phe Lys Ile Ile Arg Gln Glu Pro Ser Asp Ser Pro Met
   195 200 205
   Phe Ile Ile Asn Arg Asn Thr Gly Glu Ile Arg Thr Met Asn Asn Phe
   210 215 220
   Leu Asp Arg Glu Gln Tyr Gly Gln Tyr Ala Leu Ala Val Arg Gly Ser
   225 230 235 240
   Asp Arg Asp Gly Gly Ala Asp Gly Met Ser Ala Glu Cys Glu Cys Asn
   245 250 255
   Ile Lys Ile Leu Asp Val Asn Asp Asn Ile Pro Tyr Met Glu Gln Ser
   260 265 270
   Ser Tyr Thr Ile Glu Ile Gln Glu Asn Thr Leu Asn Ser Asn Leu Leu
   275 280 285
   Glu Ile Arg Val Ile Asp Leu Asp Glu Glu Phe Ser Ala Asn Trp Met
   290 295 300
   Ala Val Ile Phe Phe Ile Ser Gly Asn Glu Gly Asn Trp Phe Glu Ile
   305 310 315 320
   Glu Met Asn Glu Arg Thr Asn Val Gly Ile Leu Lys Val Val Lys Pro
   325 330 335
   Leu Asp Tyr Glu Ala Met Gln Ser Leu Gln Leu Ser Ile Gly Val Arg
   340 345 350
   Asn Lys Ala Glu Phe His His Ser Ile Met Ser Gln Tyr Lys Leu Lys
   355 360 365
   Ala Ser Ala Ile Ser Val Thr Val Leu Asn Val Ile Glu Gly Pro Val
   370 375 380
   Phe Arg Pro Gly Ser Lys Thr Tyr Val Val Thr Gly Asn Met Gly Ser
   385 390 395 400
   Asn Asp Lys Val Gly Asp Phe Val Ala Thr Asp Leu Asp Thr Gly Arg
   405 410 415
   Pro Ser Thr Thr Val Arg Tyr Val Met Gly Asn Asn Pro Ala Asp Leu
   420 425 430
   Leu Ala Val Asp Ser Arg Thr Gly Lys Leu Thr Leu Lys Asn Lys Val
   435 440 445
   Thr Lys Glu Gln Tyr Asn Met Leu Gly Gly Lys Tyr Gln Gly Thr Ile
   450 455 460
   Leu Ser Ile Asp Asp Asn Leu Gln Arg Thr Cys Thr Gly Thr Ile Asn
   465 470 475 480
   Ile Asn Ile Gln Ser Phe Gly Asn Asp Asp Arg Thr Asn Thr Glu Pro
   485 490 495
   Asn Thr Lys Ile Thr Thr Asn Thr Gly Arg Gln Glu Ser Thr Ser Ser
   500 505 510
   Thr Asn Tyr Asp Thr Ser Thr Thr Ser Thr Asp Ser Ser Gln Val Tyr
           515                     520                     525
   Ser Ser Glu Pro Gly Asn Gly Ala Lys Asp Leu Leu Ser Asp Asn Val
       530                     535                     540
   His Phe Gly Pro Ala Gly Ile Gly Leu Leu Ile Met Gly Phe Leu Val
   545                     550                     555                     560
   Leu Gly Leu Val Pro Phe Leu Met Ile Cys Cys Asp Cys Gly Gly Ala
                   565                     570                     575
   Pro Arg Ser Ala Ala Gly Phe Glu Pro Val Pro Glu Cys Ser Asp Gly
               580                     585                     590
   Ala Ile His Ser Trp Ala Val Glu Gly Pro Gln Pro Glu Pro Arg Asp
           595                     600                     605
   Ile Thr Thr Val Ile Pro Gln Ile Pro Pro Asp Asn Ala Asn Ile Ile
       610                     615                     620
   Glu Cys Ile Asp Asn Ser Gly Val Tyr Thr Asn Glu Tyr Gly Gly Arg
   625                     630                     635                     640
   Glu Met Gln Asp Leu Gly Gly Gly Glu Arg Met Thr Gly Phe Glu Leu
                   645                     650                     655
   Thr Glu Gly Val Lys Thr Ser Gly Met Pro Glu Ile Cys Gln Glu Tyr
               660                     665                     670
   Ser Gly Thr Leu Arg Arg Asn Ser Met Arg Glu Cys Arg Glu Gly Gly
           675                     680                     685
   Leu Asn Met Asn Phe Met Glu Ser Tyr Phe Cys Gln Lys Ala Tyr Ala
       690                     695                     700
   Tyr Ala Asp Glu Asp Glu Gly Arg Pro Ser Asn Asp Cys Leu Leu Ile
   705                     710                     715                     720
   Tyr Asp Ile Glu Gly Val Gly Ser Pro Ala Gly Ser Val Gly Cys Cys
                   725                     730                     735
   Ser Phe Ile Gly Glu Asp Leu Asp Asp Ser Phe Leu Asp Thr Leu Gly
               740                     745                     750
   Pro Lys Phe Lys Lys Leu Ala Asp Ile Ser Leu Gly Lys Glu Ser Tyr
           755                     760                     765
   Pro Asp Leu Asp Pro Ser Trp Pro Pro Gln Ser Thr Glu Pro Val Cys
       770                     775                     780
   Leu Pro Gln Glu Thr Glu Pro Val Val Ser Gly His Pro Pro Ile Ser
   785                     790                     795                     800
   Pro His Phe Gly Thr Thr Thr Val Ile Ser Glu Ser Thr Tyr Pro Ser
                   805                     810                     815
   Gly Pro Gly Val Leu His Pro Lys Pro Ile Leu Asp Pro Leu Gly Tyr
               820                     825                     830
   Gly Asn Val Thr Val Thr Glu Ser Tyr Thr Thr Ser Asp Thr Leu Lys
           835                     840                     845
   Pro Ser Val His Val His Asp Asn Arg Pro Ala Ser Asn Val Val Val
       850                     855                     860
   Thr Glu Arg Val Val Gly Pro Ile Ser Gly Ala Asp Leu His Gly Met
   865                     870                     875                     880
   Leu Glu Met Pro Asp Leu Arg Asp Gly Ser Asn Val Ile Val Thr Glu
                   885                     890                     895
   Arg Val Ile Ala Pro Ser Ser Ser Leu Pro Thr Ser Leu Thr Ile His
               900                     905                     910
   His Pro Arg Glu Ser Ser Asn Val Val Val Thr Glu Arg Val Ile Gln
           915                     920                     925
   Pro Thr Ser Gly Met Ile Gly Ser Leu Ser Met His Pro Glu Leu Ala
       930                     935                     940
   Asn Ala His Asn Val Ile Val Thr Glu Arg Val Val Ser Gly Ala Gly
   945                     950                     955                     960
   Val Thr Gly Ile Ser Gly Thr Thr Gly Ile Ser Gly Gly Ile Gly Ser
                   965                     970                     975
   Ser Gly Leu Val Gly Thr Ser Met Gly Ala Gly Ser Gly Ala Leu Ser
               980                     985                     990
   Gly Ala Gly Ile Ser Gly Gly Gly Ile Gly Leu Ser Ser Leu Gly Gly
           995                     1000                    1005
   Thr Ala Ser Ile Gly His Met Arg Ser Ser Ser Asp His His Phe Asn
       1010                1015                1020
   Gln Thr Ile Gly Ser Ala Ser Pro Ser Thr Ala Arg Ser Arg Ile Thr
   1025                1030                1035                1040
   Lys Tyr Ser Thr Val Gln Tyr Ser Lys
                   1045
<210> 3
   <211> 1117
   <212> PRT
   <213> Homo Sapien
<220>
   <221> DOMAIN
   <222> (49)...(608)
   <223> Extracellular domain
<400> 3
   Met Ala Arg Thr Arg Asp Arg Val Arg Leu Leu Leu Leu Leu Ile Cys
   1               5                   10                  15
   Phe Asn Val Gly Ser Gly Leu His Leu Gln Val Leu Ser Thr Arg Asn
               20                  25                  30
   Glu Asn Lys Leu Leu Pro Lys His Pro His Leu Val Arg Gln Lys Arg
           35                  40                  45
   Ala Trp Ile Thr Ala Pro Val Ala Leu Arg Glu Gly Glu Asp Leu Ser
       50                  55                  60
   Lys Lys Asn Pro Ile Ala Lys Ile His Ser Asp Leu Ala Glu Glu Arg
   65                  70                  75                  80
   Gly Leu Lys Ile Thr Tyr Lys Tyr Thr Gly Lys Gly Ile Thr Glu Pro
                   85                  90                  95
   Pro Phe Gly Ile Phe Val Phe Asn Lys Asp Thr Gly Glu Leu Asn Val
               100                 105                 110
   Thr Ser Ile Leu Asp Arg Glu Glu Thr Pro Phe Phe Leu Leu Thr Gly
           115                 120                 125
   Tyr Ala Leu Asp Ala Arg Gly Asn Asn Val Glu Lys Pro Leu Glu Leu
       130                 135                 140
   Arg Ile Lys Val Leu Asp Ile Asn Asp Asn Glu Pro Val Phe Thr Gln
   145                 150                 155                 160
   Asp Val Phe Val Gly Ser Val Glu Glu Leu Ser Ala Ala His Thr Leu
                   165                 170                 175
   Val Met Lys Ile Asn Ala Thr Asp Ala Asp Glu Pro Asn Thr Leu Asn
               180                 185                 190
   Ser Lys Ile Ser Tyr Arg Ile Val Ser Leu Glu Pro Ala Tyr Pro Pro
           195                 200                 205
   Val Phe Tyr Leu Asn Lys Asp Thr Gly Glu Ile Tyr Thr Thr Ser Val
       210                 215                 220
   Thr Leu Asp Arg Glu Glu His Ser Ser Tyr Thr Leu Thr Val Glu Ala
   225                 230                 235                 240
   Arg Asp Gly Asn Gly Glu Val Thr Asp Lys Pro Val Lys Gln Ala Gln
                   245                 250                 255
   Val Gln Ile Arg Ile Leu Asp Val Asn Asp Asn Ile Pro Val Val Glu
               260                 265                 270
   Asn Lys Val Leu Glu Gly Met Val Glu Glu Asn Gln Val Asn Val Glu
           275                 280                 285
   Val Thr Arg Ile Lys Val Phe Asp Ala Asp Glu Ile Gly Ser Asp Asn
       290                 295                 300
   Trp Leu Ala Asn Phe Thr Phe Ala Ser Gly Asn Glu Gly Gly Tyr Phe
   305                 310                 315                 320
   His Ile Glu Thr Asp Ala Gln Thr Asn Glu Gly Ile Val Thr Leu Ile
                   325                 330                 335
   Lys Glu Val Asp Tyr Glu Glu Met Lys Asn Leu Asp Phe Ser Val Ile
   340 345 350
   Val Ala Asn Lys Ala Ala Phe His Lys Ser Ile Arg Ser Lys Tyr Lys
   355 360 365
   Pro Thr Pro Ile Pro Ile Lys Val Lys Val Lys Asn Val Lys Glu Gly
   370 375 380
   Ile His Phe Lys Ser Ser Val Ile Ser Ile Tyr Val Ser Glu Ser Met
   385 390 395 400
   Asp Arg Ser Ser Lys Gly Gln Ile Ile Gly Asn Phe Gln Ala Phe Asp
   405 410 415
   Glu Asp Thr Gly Leu Pro Ala His Ala Arg Tyr Val Lys Leu Glu Asp
   420 425 430
   Arg Asp Asn Trp Ile Ser Val Asp Ser Val Thr Ser Glu Ile Lys Leu
   435 440 445
   Ala Lys Leu Pro Asp Phe Glu Ser Arg Tyr Val Gln Asn Gly Thr Tyr
   450 455 460
   Thr Val Lys Ile Val Ala Ile Ser Glu Asp Tyr Pro Arg Lys Thr Ile
   465 470 475 480
   Thr Gly Thr Val Leu Ile Asn Val Glu Asp Ile Asn Asp Asn Cys Pro
   485 490 495
   Thr Leu Ile Glu Pro Val Gln Thr Ile Cys His Asp Ala Glu Tyr Val
   500 505 510
   Asn Val Thr Ala Glu Asp Leu Asp Gly His Pro Asn Ser Gly Pro Phe
   515 520 525
   Ser Phe Ser Val Ile Asp Lys Pro Pro Gly Met Ala Glu Lys Trp Lys
   530 535 540
   Ile Ala Arg Gln Glu Ser Thr Ser Val Leu Leu Gln Gln Ser Glu Lys
   545 550 555 560
   Lys Leu Gly Arg Ser Glu Ile Gln Phe Leu Ile Ser Asp Asn Gln Gly
   565 570 575
   Phe Ser Cys Pro Glu Lys Gln Val Leu Thr Leu Thr Val Cys Glu Val
   580 585 590
   Leu His Gly Ser Gly Cys Arg Glu Ala Gln His Asp Ser Tyr Val Gly
   595 600 605
   Leu Gly Pro Ala Ala Ile Ala Leu Met Ile Leu Ala Phe Leu Leu Leu
   610 615 620
   Leu Leu Val Pro Leu Leu Leu Leu Met Cys His Cys Gly Lys Gly Ala
   625 630 635 640
   Lys Ala Phe Thr Pro Ile Pro Gly Thr Ile Glu Met Leu His Pro Trp
   645 650 655
   Asn Asn Glu Gly Ala Pro Pro Glu Asp Lys Val Val Pro Ser Phe Leu
   660 665 670
   Pro Val Asp Gln Gly Gly Ser Leu Val Gly Arg Asn Gly Val Gly Gly
   675 680 685
   Met Ala Lys Glu Ala Thr Met Lys Gly Ser Ser Ser Ala Ser Ile Val
   690 695 700
   Lys Gly Gln His Glu Met Ser Glu Met Asp Gly Arg Trp Glu Glu His
   705 710 715 720
   Arg Ser Leu Leu Ser Gly Arg Ala Thr Gln Phe Thr Gly Ala Thr Gly
   725 730 735
   Ala Ile Met Thr Thr Glu Thr Thr Lys Thr Ala Arg Ala Thr Gly Ala
   740 745 750
   Ser Arg Asp Met Ala Gly Ala Gln Ala Ala Ala Val Ala Leu Asn Glu
   755 760 765
   Glu Phe Leu Arg Asn Tyr Phe Thr Asp Lys Ala Ala Ser Tyr Thr Glu
   770 775 780
   Glu Asp Glu Asn His Thr Ala Lys Asp Cys Leu Leu Val Tyr Ser Gln
   785 790 795 800
   Glu Glu Thr Glu Ser Leu Asn Ala Ser Ile Gly Cys Cys Ser Phe Ile
   805 810 815
   Glu Gly Glu Leu Asp Asp Arg Phe Leu Asp Asp Leu Gly Leu Lys Phe
   820 825 830
   Lys Thr Leu Ala Glu Val Cys Leu Gly Gln Lys Ile Asp Ile Asn Lys
   835 840 845
   Glu Ile Glu Gln Arg Gln Lys Pro Ala Thr Glu Thr Ser Met Asn Thr
   850 855 860
   Ala Ser His Ser Leu Cys Glu Gln Thr Met Val Asn Ser Glu Asn Thr
   865 870 875 880
   Tyr Ser Ser Gly Ser Ser Phe Pro Val Pro Lys Ser Leu Gln Glu Ala
   885 890 895
   Asn Ala Glu Lys Val Thr Gln Glu Ile Val Thr Glu Arg Ser Val Ser
   900 905 910
   Ser Arg Gln Ala Gln Lys Val Ala Thr Pro Leu Pro Asp Pro Met Ala
   915 920 925
   Ser Arg Asn Val Ile Ala Thr Glu Thr Ser Tyr Val Thr Gly Ser Thr
   930 935 940
   Met Pro Pro Thr Thr Val Ile Leu Gly Pro Ser Gln Pro Gln Ser Leu
   945 950 955 960
   Ile Val Thr Glu Arg Val Tyr Ala Pro Ala Ser Thr Leu Val Asp Gln
   965 970 975
   Pro Tyr Ala Asn Glu Gly Thr Val Val Val Thr Glu Arg Val Ile Gln
   980 985 990
   Pro His Gly Gly Gly Ser Asn Pro Leu Glu Gly Thr Gln His Leu Gln
   995 1000 1005
   Asp Val Pro Tyr Val Met Val Arg Glu Arg Glu Ser Phe Leu Ala Pro
   1010 1015 1020
   Ser Ser Gly Val Gln Pro Thr Leu Ala Met Pro Asn Ile Ala Val Gly
   1025 1030 1035 1040
   Gln Asn Val Thr Val Thr Glu Arg Val Leu Ala Pro Ala Ser Thr Leu
   1045 1050 1055
   Gln Ser Ser Tyr Gln Ile Pro Thr Glu Asn Ser Met Thr Ala Arg Asn
   1060 1065 1070
   Thr Thr Val Ser Gly Ala Gly Val Pro Gly Pro Leu Pro Asp Phe Gly
   1075 1080 1085
   Leu Glu Glu Ser Gly His Ser Asn Ser Thr Ile Thr Thr Ser Ser Thr
   1090 1095 1100
   Arg Val Thr Lys His Ser Thr Val Gln His Ser Tyr Ser
   1105 1110 1115
<210> 4
   <211> 999
   <212> PRT
   <213> Homo Sapien
<220>
   <221> DOMAIN
   <222> (50) ... (615)
   <223> Extracellular domain
<400> 4
   Met Met Gly Leu Phe Pro Arg Thr Thr Gly Ala Leu Ala Ile Phe Val
   1 5 10 15
   Val Val Ile Leu Val His Gly Glu Leu Arg Ile Glu Thr Lys Gly Gln
   20 25 30
   Tyr Asp Glu Glu Glu Met Thr Met Gln Gln Ala Lys Arg Arg Gln Lys
   35 40 45
   Arg Glu Trp Val Lys Phe Ala Lys Pro Cys Arg Glu Gly Glu Asp Asn
   50 55 60
   Ser Lys Arg Asn Pro Ile Ala Lys Ile Thr Ser Asp Tyr Gln Ala Thr
   65 70 75 80
   Gln Lys Ile Thr Tyr Arg Ile Ser Gly Val Gly Ile Asp Gln Pro Pro
   85 90 95
   Phe Gly Ile Phe Val Val Asp Lys Asn Thr Gly Asp Ile Asn Ile Thr
                       100                     105                     110
   Ala Ile Val Asp Arg Glu Glu Thr Pro Ser Phe Leu Ile Thr Cys Arg
                   115                     120                     125
   Ala Leu Asn Ala Gln Gly Leu Asp Val Glu Lys Pro Leu Ile Leu Thr
               130                     135                     140
   Val Lys Ile Leu Asp Ile Asn Asp Asn Pro Pro Val Phe Ser Gln Gln
   145                     150                     155                     160
   Ile Phe Met Gly Glu Ile Glu Glu Asn Ser Ala Ser Asn Ser Leu Val
                           165                     170                     175
   Met Ile Leu Asn Ala Thr Asp Ala Asp Glu Pro Asn His Leu Asn Ser
                       180                     185                     190
   Lys Ile Ala Phe Lys Ile Val Ser Gln Glu Pro Ala Gly Thr Pro Met
                   195                     200                     205
   Phe Leu Leu Ser Arg Asn Thr Gly Glu Val Arg Thr Leu Thr Asn Ser
               210                     215                     220
   Leu Asp Arg Glu Gln Ala Ser Ser Tyr Arg Leu Val Val Ser Gly Ala
   225                     230                     235                     240
   Asp Lys Asp Gly Glu Gly Leu Ser Thr Gln Cys Glu Cys Asn Ile Lys
                           245                     250                     255
   Val Lys Asp Val Asn Asp Asn Phe Pro Met Phe Arg Asp Ser Gln Tyr
                       260                     265                     270
   Ser Ala Arg Ile Glu Glu Asn Ile Leu Ser Ser Glu Leu Leu Arg Phe
                   275                     280                     285
   Gln Val Thr Asp Leu Asp Glu Glu Tyr Thr Asp Asn Trp Leu Ala Val
               290                     295                     300
   Tyr Phe Phe Thr Ser Gly Asn Glu Gly Asn Trp Phe Glu Ile Gln Thr
   305                     310                     315                     320
   Asp Pro Arg Thr Asn Glu Gly Ile Leu Lys Val Val Lys Ala Leu Asp
                           325                     330                     335
   Tyr Glu Gln Leu Gln Ser Val Lys Leu Ser Ile Ala Val Lys Asn Lys
                       340                     345                     350
   Ala Glu Phe His Gln Ser Val Ile Ser Arg Tyr Arg Val Gln Ser Thr
                   355                     360                     365
   Pro Val Thr Ile Gln Val Ile Asn Val Arg Glu Gly Ile Ala Phe Arg
               370                     375                     380
   Pro Ala Ser Lys Thr Phe Thr Val Gln Lys Gly Ile Ser Ser Lys Lys
   385                     390                     395                     400
   Leu Val Asp Tyr Ile Leu Gly Thr Tyr Gln Ala Ile Asp Glu Asp Thr
                           405                     410                     415
   Asn Lys Ala Ala Ser Asn Val Lys Tyr Val Met Gly Arg Asn Asp Gly
                       420                     425                     430
   Gly Tyr Leu Met Ile Asp Ser Lys Thr Ala Glu Ile Lys Phe Val Lys
                   435                     440                     445
   Asn Met Asn Arg Asp Ser Thr Phe Ile Val Asn Lys Thr Ile Thr Ala
               450                     455                     460
   Glu Val Leu Ala Ile Asp Glu Tyr Thr Gly Lys Thr Ser Thr Gly Thr
   465                     470                     475                     480
   Val Tyr Val Arg Val Pro Asp Phe Asn Asp Asn Cys Pro Thr Ala Val
                           485                     490                     495
   Leu Glu Lys Asp Ala Val Cys Ser Ser Ser Pro Ser Val Val Val Ser
                       500                     505                     510
   Ala Arg Thr Leu Asn Asn Arg Tyr Thr Gly Pro Tyr Thr Phe Ala Leu
                   515                     520                     525
   Glu Asp Gln Pro Val Lys Leu Pro Ala Val Trp Ser Ile Thr Thr Leu
               530                     535                     540
   Asn Ala Thr Ser Ala Leu Leu Arg Ala Gln Glu Gln Ile Pro Pro Gly
   545                     550                     555                     560
   Val Tyr His Ile Ser Leu Val Leu Thr Asp Ser Gln Asn Asn Arg Cys
                           565                     570                     575
   Glu Met Pro Arg Ser Leu Thr Leu Glu Val Cys Gln Cys Asp Asn Arg
                       580                     585                     590
   Gly Ile Cys Gly Thr Ser Tyr Pro Thr Thr Ser Pro Gly Thr Arg Tyr
   595 600 605
   Gly Arg Pro His Ser Gly Arg Leu Gly Pro Ala Ala Ile Gly Leu Leu
   610 615 620
   Leu Leu Gly Leu Leu Leu Leu Leu Leu Ala Pro Leu Leu Leu Leu Thr
   625 630 635 640
   Cys Asp Cys Gly Ala Gly Ser Thr Gly Gly Val Thr Gly Gly Phe Ile
   645 650 655
   Pro Val Pro Asp Gly Ser Glu Gly Thr Ile His Gln Trp Gly Ile Glu
   660 665 670
   Gly Ala His Pro Glu Asp Lys Glu Ile Thr Asn Ile Cys Val Pro Pro
   675 680 685
   Val Thr Ala Asn Gly Ala Asp Phe Met Glu Ser Ser Glu Val Cys Thr
   690 695 700
   Asn Thr Tyr Ala Arg Gly Thr Ala Val Glu Gly Thr Ser Gly Met Glu
   705 710 715 720
   Met Thr Thr Lys Leu Gly Ala Ala Thr Glu Ser Gly Gly Ala Ala Gly
   725 730 735
   Phe Ala Thr Gly Thr Val Ser Gly Ala Ala Ser Gly Phe Gly Ala Ala
   740 745 750
   Thr Gly Val Gly Ile Cys Ser Ser Gly Gln Ser Gly Thr Met Arg Thr
   755 760 765
   Arg His Ser Thr Gly Gly Thr Asn Lys Asp Tyr Ala Asp Gly Ala Ile
   770 775 780
   Ser Met Asn Phe Leu Asp Ser Tyr Phe Ser Gln Lys Ala Phe Ala Cys
   785 790 795 800
   Ala Glu Glu Asp Asp Gly Gln Glu Ala Asn Asp Cys Leu Leu Ile Tyr
   805 810 815
   Asp Asn Glu Gly Ala Asp Ala Thr Gly Ser Pro Val Gly Ser Val Gly
   820 825 830
   Cys Cys Ser Phe Ile Ala Asp Asp Leu Asp Asp Ser Phe Leu Asp Ser
   835 840 845
   Leu Gly Pro Lys Phe Lys Lys Leu Ala Glu Ile Ser Leu Gly Val Asp
   850 855 860
   Gly Glu Gly Lys Glu Val Gln Pro Pro Ser Lys Asp Ser Gly Tyr Gly
   865 870 875 880
   Ile Glu Ser Cys Gly His Pro Ile Glu Val Gln Gln Thr Gly Phe Val
   885 890 895
   Lys Cys Gln Thr Leu Ser Gly Ser Gln Gly Ala Ser Ala Leu Ser Ala
   900 905 910
   Ser Gly Ser Val Gln Pro Ala Val Ser Ile Pro Asp Pro Leu Gln His
   915 920 925
   Gly Asn Tyr Leu Val Thr Glu Thr Tyr Ser Ala Ser Gly Ser Leu Val
   930 935 940
   Gln Pro Ser Thr Ala Gly Phe Asp Pro Leu Leu Thr Gln Asn Val Ile
   945 950 955 960
   Val Thr Glu Arg Val Ile Cys Pro Ile Ser Ser Val Pro Gly Asn Leu
   965 970 975
   Ala Gly Pro Thr Gln Leu Arg Gly Ser His Thr Met Leu Cys Thr Glu
   980 985 990
   Asp Pro Cys Ser Arg Leu Ile
   995
<210> 5
   <211> 896
   <212> PRT
   <213> Homo Sapien
<220>
   <221> DOMAIN
   <222> (136)...(690)
   <223> Extracellular domain
<400> 5
   Met Ala Ala Ala Gly Pro Arg Arg Ser Val Arg Gly Ala Val Cys Leu
    1               5                  10                  15
   His Leu Leu Leu Thr Leu Val Ile Phe Ser Arg Ala Gly Glu Ala Cys
               20                  25                  30
   Lys Lys Val Ile Leu Asn Val Pro Ser Lys Leu Glu Ala Asp Lys Ile
           35                  40                  45
   Ile Gly Arg Val Asn Leu Glu Glu Cys Phe Arg Ser Ala Asp Leu Ile
       50                  55                  60
   Arg Ser Ser Asp Pro Asp Phe Arg Val Leu Asn Asp Gly Ser Val Tyr
   65                  70                  75                  80
   Thr Ala Arg Ala Val Ala Leu Ser Asp Lys Lys Arg Ser Phe Thr Ile
                   85                  90                  95
   Trp Leu Ser Asp Lys Arg Lys Gln Thr Gln Lys Glu Val Thr Val Leu
               100                 105                 110
   Leu Glu His Gln Lys Lys Val Ser Lys Thr Arg His Thr Arg Glu Thr
           115                 120                 125
   Val Leu Arg Arg Ala Lys Arg Arg Trp Ala Pro Ile Pro Cys Ser Met
       130                 135                 140
   Gln Glu Asn Ser Leu Gly Pro Phe Pro Leu Phe Leu Gln Gln Val Glu
   145                 150                 155                 160
   Ser Asp Ala Ala Gln Asn Tyr Thr Val Phe Tyr Ser Ile Ser Gly Arg
                   165                 170                 175
   Gly Val Asp Lys Glu Pro Leu Asn Leu Phe Tyr Ile Glu Arg Asp Thr
               180                 185                 190
   Gly Asn Leu Phe Cys Thr Arg Pro Val Asp Arg Glu Glu Tyr Asp Val
           195                 200                 205
   Phe Asp Leu Ile Ala Tyr Ala Ser Thr Ala Asp Gly Tyr Ser Ala Asp
       210                 215                 220
   Leu Pro Leu Pro Leu Pro Ile Arg Val Glu Asp Glu Asn Asp Asn His
   225                 230                 235                 240
   Pro Val Phe Thr Glu Ala Ile Tyr Asn Phe Glu Val Leu Glu Ser Ser
                   245                 250                 255
   Arg Pro Gly Thr Thr Val Gly Val Val Cys Ala Thr Asp Arg Asp Glu
               260                 265                 270
   Pro Asp Thr Met His Thr Arg Leu Lys Tyr Ser Ile Leu Gln Gln Thr
           275                 280                 285
   Pro Arg Ser Pro Gly Leu Phe Ser Val His Pro Ser Thr Gly Val Ile
       290                 295                 300
   Thr Thr Val Ser His Tyr Leu Asp Arg Glu Val Val Asp Lys Tyr Ser
   305                 310                 315                 320
   Leu Ile Met Lys Val Gln Asp Met Asp Gly Gln Phe Phe Gly Leu Ile
                   325                 330                 335
   Gly Thr Ser Thr Cys Ile Ile Thr Val Thr Asp Ser Asn Asp Asn Ala
               340                 345                 350
   Pro Thr Phe Arg Gln Asn Ala Tyr Glu Ala Phe Val Glu Glu Asn Ala
           355                 360                 365
   Phe Asn Val Glu Ile Leu Arg Ile Pro Ile Glu Asp Lys Asp Leu Ile
       370                 375                 380
   Asn Thr Ala Asn Trp Arg Val Asn Phe Thr Ile Leu Lys Gly Asn Glu
   385                 390                 395                 400
   Asn Gly His Phe Lys Ile Ser Thr Asp Lys Glu Thr Asn Glu Gly Val
                   405                 410                 415
   Leu Ser Val Val Lys Pro Leu Asn Tyr Glu Glu Asn Arg Gln Val Asn
               420                 425                 430
   Leu Glu Ile Gly Val Asn Asn Glu Ala Pro Phe Ala Arg Asp Ile Pro
           435                 440                 445
   Arg Val Thr Ala Leu Asn Arg Ala Leu Val Thr Val His Val Arg Asp
       450                 455                 460
   Leu Asp Glu Gly Pro Glu Cys Thr Pro Ala Ala Gln Tyr Val Arg Ile
   465 470 475 480
   Lys Glu Asn Leu Ala Val Gly Ser Lys Ile Asn Gly Tyr Lys Ala Tyr
   485 490 495
   Asp Pro Glu Asn Arg Asn Gly Asn Gly Leu Arg Tyr Lys Lys Leu His
   500 505 510
   Asp Pro Lys Gly Trp Ile Thr Ile Asp Glu Ile Ser Gly Ser Ile Ile
   515 520 525
   Thr Ser Lys Ile Leu Asp Arg Glu Val Glu Thr Pro Lys Asn Glu Leu
   530 535 540
   Tyr Asn Ile Thr Val Leu Ala Ile Asp Lys Asp Asp Arg Ser Cys Thr
   545 550 555 560
   Gly Thr Leu Ala Val Asn Ile Glu Asp Val Asn Asp Asn Pro Pro Glu
   565 570 575
   Ile Leu Gln Glu Tyr Val Val Ile Cys Lys Pro Lys Met Gly Tyr Thr
   580 585 590
   Asp Ile Leu Ala Val Asp Pro Asp Glu Pro Val His Gly Ala Pro Phe
   595 600 605
   Tyr Phe Ser Leu Pro Asn Thr Ser Pro Glu Ile Ser Arg Leu Trp Ser
   610 615 620
   Leu Thr Lys Val Asn Asp Thr Ala Ala Arg Leu Ser Tyr Gln Lys Asn
   625 630 635 640
   Ala Gly Phe Gln Glu Tyr Thr Ile Pro Ile Thr Val Lys Asp Arg Ala
   645 650 655
   Gly Gln Ala Ala Thr Lys Leu Leu Arg Val Asn Leu Cys Glu Cys Thr
   660 665 670
   His Pro Thr Gln Cys Arg Ala Thr Ser Arg Ser Thr Gly Val Ile Leu
   675 680 685
   Gly Lys Trp Ala Ile Leu Ala Ile Leu Leu Gly Ile Ala Leu Leu Phe
   690 695 700
   Ser Val Leu Leu Thr Leu Val Cys Gly Val Phe Gly Ala Thr Lys Gly
   705 710 715 720
   Lys Arg Phe Pro Glu Asp Leu Ala Gln Gln Asn Leu Ile Ile Ser Asn
   725 730 735
   Thr Glu Ala Pro Gly Asp Asp Arg Val Cys Ser Ala Asn Gly Phe Met
   740 745 750
   Thr Gln Thr Thr Asn Asn Ser Ser Gln Gly Phe Cys Gly Thr Met Gly
   755 760 765
   Ser Gly Met Lys Asn Gly Gly Gln Glu Thr Ile Glu Met Met Lys Gly
   770 775 780
   Gly Asn Gln Thr Leu Glu Ser Cys Arg Gly Ala Gly His His His Thr
   785 790 795 800
   Leu Asp Ser Cys Arg Gly Gly His Thr Glu Val Asp Asn Cys Arg Tyr
   805 810 815
   Thr Tyr Ser Glu Trp His Ser Phe Thr Gln Pro Arg Leu Gly Glu Lys
   820 825 830
   Leu His Arg Cys Asn Gln Asn Glu Asp Arg Met Pro Ser Gln Asp Tyr
   835 840 845
   Val Leu Thr Tyr Asn Tyr Glu Gly Arg Gly Ser Pro Ala Gly Ser Val
   850 855 860
   Gly Cys Cys Ser Glu Lys Gln Glu Glu Asp Gly Leu Asp Phe Leu Asn
   865 870 875 880
   Asn Leu Glu Pro Lys Phe Ile Thr Leu Ala Glu Ala Cys Thr Lys Arg
   885 890 895
<210> 6
   <211> 31
   <212> PRT
   <213> Influenza Strain A
<220>
   <223> HMIVF
<400> 6
   Asp Lys Ile Cys Leu Gly His His Ala Val Ser Asn Gly Thr Lys Val
   1 5 10 15
   Asn Thr Leu Thr Glu Arg Gly Val Glu Val Val Asn Ala Thr Glu
   20 25 30
<210> 7
   <211> 41
   <212> PRT
   <213> Influenza Strain A
<220>
   <223> HMIVH
<400> 7
   Gln Asp Leu Pro Gly Asn Asp Asn Asn Thr Ala Thr Leu Cys Leu Gly
   1 5 10 15
   His His Ala Val Pro Asn Gly Thr Leu Val Lys Thr Ile Thr Asp Asp
   20 25 30
   Gln Ile Glu Val Thr Asn Ala Thr Glu
   35 40
<210> 8
   <211> 41
   <212> PRT
   <213> Influenza Strain A
<220>
   <223> HMIV6
<400> 8
   Gln Asn Leu Pro Gly Asn Asp Asn Ser Thr Ala Thr Leu Cys Leu Ala
   1 5 10 15
   His His Ala Val Pro Asn Gly Thr Leu Val Lys Thr Ile Thr Asn Asp
   20 25 30
   Gln Ile Glu Val Thr Asn Ala Thr Glu
   35 40
<210> 9
   <211> 42
   <212> PRT
   <213> Influenza Strain A
<220>
   <223> HMIVV
<400> 9
   Gln Asp Leu Pro Gly Asn Asp Asn Asn Ser Thr Ala Thr Leu Cys Leu
   1 5 10 15
   Gly His His Ala Val Pro Asn Gly Thr Leu Val Lys Thr Ile Thr Asn
   20 25 30
   Asp Gln Ile Glu Val Thr Asn Ala Thr Glu
   35 40
<210> 10
   <211> 41
   <212> PRT
   <213> Influenza Strain A
<220>
   <223> HMIVDU
<400> 10
   Gln Asp Leu Pro Gly Asn Asp Asn Ser Thr Ala Thr Leu Cys Leu Gly
   1 5 10 15
   His His Ala Val Pro Asn Gly Thr Ile Val Lys Thr Ile Thr Asp Asp
   20 25 30
   Gln Ile Glu Val Thr Asn Ala Thr Glu
   35 40
<210> 11
   <211> 41
   <212> PRT
   <213> Influenza Strain A
<220>
   <223> HMIVH3
<400> 11
   Gln Tyr Leu Pro Gly Asn Asp Asn Ser Thr Ala Thr Leu Cys Leu Gly
   1 5 10 15
   His His Ala Val Pro Asn Gly Thr Leu Val Lys Thr Ile Thr Asn Asp
   20 25 30
   Gln Ile Glu Val Thr Asn Ala Thr Glu
   35 40
<210> 12
   <211> 31
   <212> PRT
   <213> Influenza Strain A
<220>
   <223> HMIVH7
<400> 12
   Asp Lys Ile Cys Leu Gly His His Ala Val Ala Asn Gly Thr Lys Val
   1 5 10 15
   Asn Thr Leu Thr Glu Arg Gly Ile Glu Val Val Asn Ala Thr Glu
   20 25 30
<210> 13
   <211> 31
   <212> PRT
   <213> Influenza Strain A
<220>
   <223> HMIV10
<400> 13
   Asp Lys Ile Cys Leu Gly His His Ala Val Pro Asn Gly Ile Ile Val
   1 5 10 15
   Lys Thr Leu Thr Asn Glu Lys Glu Glu Val Thr Asn Ala Thr Glu
   20 25 30
<210> 14
   <211> 35
   <212> PRT
   <213> Influenza Strain A
<220>
   <223> HMIVH6
<400> 14
   Asp Lys Ile Cys Ile Gly Tyr His Ala Asn Asn Ser Thr Thr Gln Ile
   1 5 10 15
   Asp Thr Ile Leu Glu Lys Asn Val Thr Val Thr His Ser Val Glu Leu
   20 25 30
   Leu Glu Asn
   35
<210> 15
   <211> 35
   <212> PRT
   <213> Influenza Strain A
<220>
   <223> HMIV5
<400> 15
   Asp Thr Ile Cys Ile Gly Tyr His Ala Asn Asn Ser Thr Asp Thr Val
   1 5 10 15
   Asp Thr Ile Phe Glu Lys Asn Val Ala Val Thr His Ser Val Asn Leu
   20 25 30
   Leu Glu Asp
   35
<210> 16
   <211> 35
   <212> PRT
   <213> Influenza Strain A
<220>
   <223> HMIV
<400> 16
   Asp Thr Ile Cys Ile Gly Tyr His Ala Asn Asn Ser Thr Asp Thr Val
   1 5 10 15
   Asp Thr Val Leu Glu Lys Asn Val Thr Val Thr His Ser Val Asn Leu
   20 25 30
   Leu Glu Asp
   35
<210> 17
   <211> 35
   <212> PRT
   <213> Influenza Strain A
<220>
   <223> HMIVUR
<400> 17
   Asp Thr Ile Cys Ile Gly Tyr His Ala Asn Asn Ser Thr Asp Thr Val
   1 5 10 15
   Asp Thr Val Leu Glu Lys Asn Val Thr Val Thr His Ser Val Asn Leu
   20 25 30
   Leu Glu Asp
   35
<210> 18
   <211> 35
   <212> PRT
   <213> Influenza Strain A
<220>
   <223> HMIV2
<400> 18
   Asp Gln Ile Cys Ile Gly Tyr His Ala Asn Asn Ser Thr Glu Lys Val
   1 5 10 15
   Asp Thr Asn Leu Glu Arg Asn Val Thr Val Thr His Ala Lys Asp Ile
   20 25 30
   Leu Glu Lys
   35
<210> 19
   <211> 35
   <212> PRT
   <213> Influenza Strain A
<220>
   <223> HMIVH2
<400> 19
   Asp Gln Ile Cys Ile Gly Tyr His Ala Asn Asn Ser Thr Glu Lys Val
   1 5 10 15
   Asp Thr Ile Leu Glu Arg Asn Val Thr Val Thr His Ala Lys Asp Ile
   20 25 30
   Leu Glu Lys
   35
<210> 20
   <211> 35
   <212> PRT
   <213> Influenza Strain A
<220>
   <223> HMIVH5
<400> 20
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys
   35
<210> 21
   <211> 35
   <212> PRT
   <213> Influenza Strain A
<220>
   <223> HMIVN1
<400> 21
   Asp Thr Leu Cys Ile Gly Tyr His Ala Asn Asn Ser Thr Asp Thr Val
   1 5 10 15
   Asp Thr Val Leu Glu Lys Asn Val Thr Val Thr His Ser Val Asn Leu
   20 25 30
   Leu Glu Asp
   35
<210> 22
   <211> 12
   <212> PRT
   <213> Chicken bFGF-R
<400> 22
   Glu Lys Met Glu Lys Lys Leu His Ala Val Pro Ala
   1 5 10
<210> 23
   <211> 12
   <212> PRT
   <213> Mouse FGF-R
<220>
   <223> i
<400> 23
   Glu Lys Met Glu Lys Lys Leu His Ala Val Pro Ala
   1 5 10
<210> 24
   <211> 12
   <212> PRT
   <213> Frog FGF-R
<220>
   <223> i
<400> 24
   Glu Lys Met Glu Lys Lys Leu His Ala Val Pro Ala
   1 5 10
<210 > 25
   <211> 12
   <212> PRT
   <213> Mouse KGF-R
<220>
   <223> i
<400> 25
   Glu Lys Met Glu Lys Arg Leu His Ala Val Pro Ala
   1 5 10